# EUROPEAN PATENT SPECIFICATION

(11) **EP 4 339 183 B1**
(45) Date of publication and mention of the grant of the patent: **15.04.2026**
(21) Application number: 22807545.3
(22) Date of filing: 12.05.2022
(51) Int. Cl.: C07C 51/44, C07C 51/48, C07C 53/02, C07C 51/46

(54) **METHOD FOR COLLECTING CARBOXYLIC ACID**
VERFAHREN ZUM SAMMELN VON CARBONSÄURE
PROCÉDÉ DE COLLECTE D'ACIDE CARBOXYLIQUE

(30) Priority: 14.05.2021 JP 2021082778
(43) Date of publication of application: 20.03.2024
(73) Proprietor: Refine Holdings Co., Ltd., Anpachi-gun, Gifu 503-0212 (JP); Spiber Inc., Tsuruoka-shi, Yamagata 997-0052 (JP)
(72) Inventor: FUJITA Tomoya, Tokyo 100-0005 (JP); IWAFUNE Mitsuhiro, Tokyo 100-0005 (JP); SUZUKI Takao, Tokyo 100-0005 (JP); TAKEYAMA Tomokiyo, Tokyo 100-0005 (JP); ISHIZAWA Hideki, Tokyo 100-0005 (JP); ODA Akiyoshi, Tokyo 100-0005 (JP); TAKAHASHI Hiroaki, Tokyo 100-0005 (JP); OHNO Junya, Tokyo 100-0005 (JP); NAKASHIMA Kouji, Tokyo 100-0005 (JP); NOUMI Tadashi, Tokyo 100-0005 (JP); ISHII Hideto, Tsuruoka-shi, Yamagata 997-0052 (JP)
(74) Representative: Dehns
(86) International application number: PCT/JP2022/020120
(87) International publication number: WO 2022/239847

(56) References cited:
- WO-A1-2020/108066
- JP-A- 2003 520 832
- JP-A- 2003 524 640
- JP-A- H09 151 158
- JP-A- H11 228 486
- JP-A- S5 724 324

## Description

### Technical Field

The present disclosure relates to a method for recovering a carboxylic acid. Specifically, the present disclosure relates to, for example, a method for recovering a carboxylic acid as a carboxylic acid anhydride from an aqueous solution containing the carboxylic acid and inorganic salts.

### Background Art

For example, aqueous solutions of carboxylic acids such as formic acid or acetic acid result as wastewater in many industrially important various production or treatment processes. In recent years, it is required to clean such a waste aqueous solution from the viewpoint of increasing interest in environmental pollution and strengthening various regulations, and it is required to recover a carboxylic acid contained as a process by-product from the viewpoint of reusing economically valuable resources.

As a method for recovering a carboxylic acid from a solution, not only the treatment of wastewater as described above, but also, for example, a considerable number of treatment methods have been conventionally proposed in view of a purification technique of a dilute carboxylic acid aqueous solution obtained by production of a lower fatty acid by microorganisms in a fermentation industry.

It is known to use an organic solvent as a method for extracting carboxylic acid from a dilute aqueous solution. However, a lower carboxylic acid such as formic acid or acetic acid has a high affinity with water, and a significantly large number of compounds have been tried as extractants, but sufficiently satisfactory results have not been obtained. That is, since a partition coefficient that may affect greatly the efficiency of the extraction method is generally small with respect to the lower fatty acid, to increase extraction efficiency, the amount of extractant used inevitably has to be increased and energy consumption in a separation step has to become increased.

For example, Patent Literature 1 discloses a method for purifying acetic acid in which an acetic acid aqueous solution having a concentration of acetic acid in a range of 10 wt% to 50 wt% is introduced into an extraction device as a raw material liquid, an extractant containing isopropyl acetate in a range of 0.6 times to 3.0 times the weight of the raw material liquid is supplied to the extraction device and is brought into contact with the raw material liquid, acetic acid is extracted into an extractant phase and is separated into an extraction liquid and a raffinate liquid, the extraction liquid is supplied to an azeotropic distillation tower, and dehydrated purified acetic acid is recovered through a bottom of the azeotropic distillation tower.

In addition, Patent Literature 2 discloses a method for preparing an organic carboxylic acid-containing aqueous solution rich in an organic carboxylic acid by concentrating an organic carboxylic acid-containing aqueous solution as a raw material, the method including: a step (a) of bringing an extraction solvent into contact with an organic carboxylic acid-containing aqueous solution as a raw material to extract the organic carboxylic acid into an extraction phase; a step (b) of separating the extraction phase obtained from the step (a) into a fraction rich in the extraction solvent and a fraction rich in the organic carboxylic acid; and a step (c) of separating a raffinate phase discharged from the step (a) into a fraction rich in the extraction solvent and a fraction enriched rich in water.

In the methods described in Patent Literatures 1 and 2, an ether, a ketone, a carboxylic acid ester, or the like, which is a lower boiling point component than the targeted carboxylic acid, is used as an extractant. In a liquid-liquid extraction device, the target carboxylic acid is extracted in the fraction rich in the extractant. Therefore, when the extractant has a low boiling point, it is required to heat the entire amount of the extractant in a distillation and purification step, and thus an energy load is large. For example, ethyl acetate is a commonly used extraction solvent among organic solvents because it has a relatively large partition coefficient for a lower fatty acid, in particular, acetic acid, and is easily available. However, since ethyl acetate has a boiling point lower than that of acetic acid, it is required to evaporate the entire amount of the extraction solvent used in a large amount. Further, since a large amount of water is dissolved in the extraction liquid and a dissolution loss in water is large, ethyl acetate is also unsatisfactory in terms of mutual dissolution with water.

In addition, Patent Literatures 3 and 4 disclose that when a lower fatty acid is extracted from an aqueous solution containing a lower fatty acid such as formic acid, acetic acid, or propionic acid, a mixed solvent of trioctylphosphine oxide and isophorone or a mixed solvent of trioctylphosphine oxide and trimethylcyclohexanone is used as an extraction solvent.

Patent Literature 5 discloses a method for recovering a carboxylic acid from a water-soluble aqueous solution containing a carboxylic acid, the method including: a contacting step of bringing the water-soluble aqueous solution into contact with a solvent substantially formed of a mixed trialkylphosphine oxide in a countercurrent liquid-liquid extraction stream to transfer the acid from the water-soluble solution to the solvent so as to produce a raffinate having a relatively low acid content and a rich solvent having a relatively high acid content, the acid-rich solvent containing some water; a hydration step of hydrating the rich solvent by applying heat to the rich solvent and separating water therefrom to produce a water stream and a dehydrated solvent-rich stream; and a stripping step of stripping the acid from the dehydrated solvent-rich stream by applying heat to the dehydrated solvent-rich stream and producing a solvent substantially formed of a mixed trialkylphosphine oxide and an acid stream containing the acid for recirculation to the liquid-liquid extraction stream.

In addition, Patent Literatures 6 and 7 disclose a method for extracting a lower fatty acid, in which when a lower fatty acid is extracted from an aqueous solution containing a lower fatty acid such as formic acid, acetic acid, or propionic acid, a mixed solvent of a tertiary amine having a boiling point higher than that of the lower fatty acid and a branched primary alcohol having a boiling point higher than that of the lower fatty acid, such as diethylhexanol or 3,5,5-trimethylhexanol, a tertiary amine having a boiling point higher than that of the lower fatty acid, and a linear primary alcohol having a boiling point higher than that of the lower fatty acid, such as n-hexanol, n-heptanol, n-octanol, or n-nonanol, are used. A combination thereof increases the extraction rate of the carboxylic acid, but in this case, the alcohol is dissolved in water. Dissolution means that alcohol is lost during the extraction operation, and the lost amount needs to be newly added from the outside, even if the amount is small, when the carboxylic acid to be recovered is diluted, the meaning of recovery is lost.

In addition, Patent Literature 8 discloses a method for recovering an organic acid, in which an organic acid in an aqueous solution containing an organic acid is extracted by bringing the aqueous solution into contact with an organic solvent obtained by selecting one or two or more from the group of primary amines to quaternary amines and the group of phosphoric acid esters and diluting the organic solvent with a petroleum-based hydrocarbon, and then the organic solvent to which the organic acid is transferred is heated or heated and distilled, or is brought into contact with a liquid containing Na, Mg, NH₃, or the like to separate and recover the organic acid and recycle the organic solvent (A).

On the other hand, as described in Patent Literatures 3 to 8, when an extractant having a boiling point higher than that of a lower fatty acid such as a trialkylphosphine oxide or an amine compound is used, the carboxylic acid extracted in the fraction rich in the extractant can be easily separated in the distillation and purification step, but the extraction rate by the trialkylphosphine oxide or the amine compound is not high. As described in Patent Literatures 6 and 7, a combination of the amine compound and the branched primary alcohol or the linear primary alcohol increases the extraction rate of the carboxylic acid, but in this case, the alcohol is dissolved in water. Dissolution means that alcohol is lost during the extraction operation, and the lost amount needs to be newly added from the outside, even if the amount is small, when the carboxylic acid to be recovered is diluted, the meaning of recovery is lost. In addition, although a specific extraction method related to the method described in Patent Literature 8 is not described, the extraction rate is about 50%, which is not high when it is taken as an extraction equilibrium. Further, the heating distillation can be separated from the physical properties, but specific results are not shown.

Patent Literature 9 discloses a method for producing anhydrous or almost anhydrous formic acid by hydrolyzing methyl formate, separating unreacted methyl formate and generated methanol by distillation in a first distillation step, and then removing formic acid from a bottom product by liquid-liquid extraction, in which (a) water and methyl formate are used at a molar ratio of 1:1 to 30:1 during the hydrolysis of methyl formate, (b) an effluent of a hydrolysis reactor is supplied to a distillation tower, methanol and methyl formate are separated from a formic acid-water-bottom product, methyl formate and methanol are introduced into a top of the column or into another column, and a partial stream of methyl formate is recirculated downstream from an addition site of the hydrolysis mixture in the first distillation step, (c) aqueous formic acid is extracted from the bottom product in the first distillation step at a temperature of 20 to 100°C using at least a stoichiometric amount of an amine that is slightly soluble in water having a pKa-value from 4 to 9 and forms hydroformate together with an amine having a high boiling point, (d) the resulting amine extract is dehydrated in a distillation tower at a temperature of 30 to 120°C and a pressure of 10 to 400 mbar, in the steps (c) and/or (d), a hydrophobic solvent that forms a heteroazeotrope with water and formic acid or has a boiling point that is higher than those of water and formic acid but is lower than that of the amine used is added, (e) the dehydrated extract is introduced into the top tray of a decomposition column, amine hydroformate is decomposed into formic acid and an amine therein at a temperature of 110 to 240°C, in this case, formic acid and a solvent are obtained as a top product of the decomposition column, in some cases, as an azeotrope, an amine and a solvent are obtained as a bottom product, and then the top product is separated into formic acid and a solvent, and (f) the resulting methanol, methyl formate, water, amine, and solvent streams are recirculated to the process and/or treatment step, and in this case, the recirculated amine is introduced through the bottom of the decomposition column for purification in an adsorption column.

In the method of Patent Literature 9, formic acid is extracted using a high boiling point amine, and after water cutting by distillation, an aliphatic, cycloaliphatic, or aromatic hydrocarbon having 8 to 12 C-atoms is added as a hydrophobic solvent to decompose the formic acid and the amine at a high temperature to purify the formic acid. However, since the decomposition of the formic acid and the amine is performed at a high temperature, there are concerns about in-column corrosion, generation of a decomposition gas, and the like, in the distillation tower.

In addition, Patent Literature 10 discloses the extraction of formic acid with N,N-di-n-butylformamide. However, the extractant contains a significant amount of water together with formic acid (in the described example, 43 wt%, based on the extracted formic acid), and thus a large amount of water needs to be evaporated. Furthermore, formic acid is obtained in an anhydrous state by only 14%, and a residue is obtained as a product of 70%.

As described above, in Patent Literature 10, N,N-di-n-butylformamide having a high boiling point was used for formic acid, but a large amount of water was also extracted in a fraction rich in the extractant together with the carboxylic acid, and it was difficult to distill water and to recover formic anhydride even in the distillation step.

Furthermore, as an aqueous solution of a carboxylic acid generated as wastewater in various production or treatment steps, there is an aspect in which a carboxylic acid concentration is diluted to a mass fraction of about 0.01 to 0.3, and an inorganic salt is contained in the aqueous solution in a mass fraction range of 0.003 to 0.2, and in a case where the inorganic salt is present in the aqueous solution as described above, it is unclear whether the above-described method can be applied as a method for recovering a carboxylic acid.

Patent Literature 11 discloses a method for linkage recovery of organic acid in an aqueous organic acid solution, wherein a solution with an organic acid concentration lower than 20wt% is mixed with an extractant and then subjected to countercurrent extraction to obtain an extract phase and a raffinate phase; the extract phase and a solution with an organic acid concentration higher than 70wt% are jointly subjected to azeotropic distillation for extraction. The extractant is any one or a combination of at least two of an ester extractant, an organic phosphorus extractant, an organic amine extractant or a ketone extractant.

Patent Literature 12 discloses a method of extracting acetic acid from acetic acid-containing wastewater using an extraction solvent. In step A an extraction solvent and water are introduced into the extraction solvent recovery tower, and an azeotropic mixture of the extraction solvent and water are removed as a distillate from the top of the recovery tower, the azeotropic mixture which is the distillate from the extraction solvent recovery column is separated into a solvent phase and an aqueous phase. The extraction solvent is an aliphatic ketone having 6 to 10 carbon atoms.

### Citation List

### Patent Literature

Patent Literature 1: JP 9-151158 A
Patent Literature 2: JP 2018-062512 A
Patent Literature 3: JP 61-176550 A
Patent Literature 4: JP 61-176551 A
Patent Literature 5: JP 08-283191 A
Patent Literature 6: JP 61-176552 A
Patent Literature 7: JP 61-176553 A
Patent Literature 8: JP 55-154935 A
Patent Literature 9: JP 61-043133 A
Patent Literature 10: DE 2545658 A
Patent Literature 11: WO2020/108066 A1
Patent Literature 12: JP H11 228486 A

### Summary of Invention

### Technical Problem

Therefore, the present disclosure aims to provide a method for recovering a carboxylic acid that solves the problems in the related art as described above. In addition, the present disclosure relates to, for example, a method for recovering a carboxylic acid as a carboxylic acid anhydride from an aqueous solution containing a carboxylic acid and an inorganic salt. In addition, the present disclosure also aims to provide a method for recovering a carboxylic acid that has a low risk of corrosion in a device or the like and can reduce costs for a device material or the like.

### Solution to Problem

In order to solve the above problems, we, the inventors, have conducted intensive studies and investigations, and as a result, we have developed a method for recovering a carboxylic acid, in which method the carboxylic acid is separated from an aqueous solution containing water and the carboxylic acid and having a carboxylic acid concentration of 0.05 to 0.3 in terms of the mass fraction, and the separated carboxylic acid is purified and obtained as a carboxylic acid anhydride containing less than 0.01 of water and not less than 0.99 of carboxylic acid in terms of the mass fraction. The method includes:
a) a first step comprising a liquid-liquid extraction step of bringing the aqueous solution i:nto liquid-liquid contact with a prepared extractant, the prepared extractant containing a component for extracting the carboxylic acid and a diluent to dissolve the component, the liquid-liquid contact resulting in a state that, on the aqueous solution side, the component of the prepared extractant is dissolved in less than 0.001 in term of the mass fraction and the carboxylic acid concentration in the aqueous solution is less than 0.005 in term of the mass fraction, and, on the prepared extractant side, the carboxylic acid separated with a recovery rate of not less than 90% from the aqueous solution is dissolved and water dissolved in less than 0.05 in terms of the mass fraction;
b) a second step comprising a step of distilling the prepared extractant containing the carboxylic acid and water that is subjected to the first step, azeotropically distilling the diluent in the prepared extractant and water, separating the distillate into an extractant component layer mainly containing the diluent and an aqueous layer with a decanter provided in a distillation column and then discharging the aqueous layer, mixing the discharged water with a primary aqueous solution or a secondary aqueous solution in the first step or disposing of the discharged water as wastewater while returning the extractant component layer mainly containing the diluent to the distillation step as reflux, and discharging the prepared extractant containing the carboxylic acid through a bottom of the column; and
c) a third step comprising re-distilling the prepared extractant containing the carboxylic acid discharged by the second step, and

in a case where there is no azeotrope between the carboxylic acid and the diluent in the prepared extractant, discharging, through a top of the column, a purified carboxylic acid containing, in terms of the mass fraction, less than 0.01 of water and less than 0.01 of the prepared extractant, and returning, to the first step, the prepared extractant from which the carboxylic acid and water are removed, the prepared extractant being discharged through the bottom of the column,
in a case where the carboxylic acid and the diluent in the prepared extractant have a minimum boiling azeotrope, distilling the diluent and the carboxylic acid by azeotropic distillation, separating the distillate into an extractant component layer mainly containing the diluent and a carboxylic acid layer with a decanter provided in a distillation column, discharging the carboxylic acid layer containing, in terms of the mass fraction, less than 0.01 of water and less than 0.01 of the prepared extractant to obtain the purified carboxylic acid while returning the extractant component layer mainly containing the diluent to the distillation step as reflux, and returning, to the first step, the prepared extractant from which the carboxylic acid and water are removed, the prepared extractant being discharged through a bottom of the column,
   the diluent in the prepared extractant being a hydrophobic solvent having a boiling point higher than that of water and higher than that of the carboxylic acid at atmospheric pressure, which is characterized in that the diluent has a minimum boiling azeotrope with water, a water concentration in an azeotropic composition with the diluent is not less than 0.2 in terms of the mass fraction, and the diluent has no azeotrope with the carboxylic acid, or
   the diluent in the prepared extractant being a hydrophobic solvent having a boiling point higher than that of water and higher than that of the carboxylic acid at atmospheric pressure, which is characterized in that the diluent has a minimum boiling azeotrope with water, a water concentration in an azeotropic composition with the diluent is not less than 0.2 in terms of the mass fraction, the carboxylic acid has a minimum boiling azeotrope with the diluent, the carboxylic acid and the diluent are separated into layers at any ratio, and the dissolution of the diluent in the carboxylic acid is less than 0.002 in terms of the mass fraction, and
   the component for extracting the carboxylic acid being a poorly water-soluble organic solvent that has a boiling point higher than that of the diluent, has a carboxylic acid distribution ratio D of "water" to "the component for extracting" (= the carboxylic acid in the component for extracting/the carboxylic acid in water) of not less than 0.3 and is completely compatible with the diluent.

In an embodiment of the method for recovering a carboxylic acid according to the present disclosure, the aqueous solution further contains an inorganic salt in a mass fraction range of 0.003 to 0.2, and in the liquid-liquid extraction step in the first step, the inorganic salt in the aqueous solution is dissolved in the prepared extractant in an amount of less than 0.0001 and in the water in the aqueous solution in an amount of 0.003 to less than 0.2.

In an embodiment of the method for recovering a carboxylic acid according to the present disclosure, the carboxylic acid contained in the aqueous solution is selected from the group consisting of formic acid, acetic acid, and propionic acid.

In an embodiment of the method for recovering a carboxylic acid according to the present disclosure, the inorganic salt contained in the aqueous solution is selected from the group consisting of a metal chloride, a metal sulfate, a metal hydrogen sulfate, a metal hydroxide, a metal carbonate, a metal hydrogen carbonate, a metal phosphate, a metal hydrogen phosphate, and a metal borate.

In an embodiment of the method for recovering a carboxylic acid according to the present disclosure, a boiling point of the diluent in the prepared extractant is 110 to 220°C at atmospheric pressure, and is a hydrophobic solvent having a solubility in water of less than 0.001 in terms of the mass fraction at 25°C.

In an embodiment of the method for recovering a carboxylic acid according to the present disclosure, the diluent in the prepared extractant is at least one selected from the group consisting of toluene, octane, isooctane, nonane, decane, undecane, dodecane, o-xylene, m-xylene, p-xylene, and ethylbenzene.

In an embodiment of the method for recovering a carboxylic acid according to the present disclosure, the aqueous solution contains impurities that are dissolved in a carboxylic acid and are not dissolved in water, and
before applying the aqueous solution to the first step, a step of removing the impurities by filtering the aqueous solution is performed, or the carboxylic acid is recovered in the prepared extractant in the first step, such that the impurities that precipitate at an interface with an extractant phase are extracted from a nozzle provided at a top or a bottom of a liquid-liquid extraction device together with the prepared extractant and the aqueous solution, and the impurities are removed by separating a liquid containing the extracted impurities into impurities and a liquid by filtration or centrifugation and mixing a recovered liquid with the primary aqueous solution in the first step.

In an embodiment of the method for recovering a carboxylic acid according to the present disclosure, the component for extracting a carboxylic acid in the prepared extractant is selected from the group consisting of an organophosphorus compound and an amide compound.

In an embodiment of the method for recovering a carboxylic acid according to the present disclosure, a mixing ratio of the component for extracting a carboxylic acid to the diluent in the prepared extractant is a ratio of the component for extracting a carboxylic acid : the diluent of 1:5 to 9:1 in terms of the mass ratio.

In an embodiment of the method for recovering a carboxylic acid according to the present disclosure, when the aqueous solution containing the inorganic salt is subjected to the first step, a liquid-liquid extraction device is operated at a temperature of 10 to 90°C.

In an embodiment of the method for recovering a carboxylic acid according to the present disclosure, the second step is performed at a degree of reduced pressure of 6.67 to 66.7 kPa.

In an embodiment of the method for recovering a carboxylic acid according to the present disclosure, a filtering step is added to a line through which a bottom liquid is transferred in the third step to remove the inorganic salt that precipitates at the bottom of the distillation column by distilling water from the prepared extractant containing the carboxylic acid and water in the second step.

In an embodiment of the method for recovering a carboxylic acid according to the present disclosure, the second step includes a line through which the prepared extractant is supplied to the column from an arbitrary tray above a raw material supply tray, separately from the reflux of the extractant component layer mainly containing the diluent, to improve a recovery rate of the carboxylic acid.

In an embodiment of the method for recovering a carboxylic acid according to the present disclosure, the third step is performed at a degree of reduced pressure of 6.67 to 66.7 kPa.

In an embodiment of the method for recovering a carboxylic acid according to the present disclosure, the third step includes a line through which the extractant component layer mainly containing a diluent is supplied to the column from an arbitrary tray below a raw material supply tray, separately from the reflux of the extractant component layer mainly containing the diluent, to improve a recovery rate of the carboxylic acid.

In an embodiment of the method for recovering a carboxylic acid according to the present disclosure, the method further includes a fourth step of obtaining a carboxylic acid anhydride containing not less than 0.99 of a carboxylic acid and not more than 0.002 of water from the top of the distillation column or the middle of the distillation column by re-distilling the carboxylic acid containing, in terms of the mass fraction, less than 0.01 of water and less than 0.01 of a prepared extractant component, and distilling water and the prepared extractant component, the carboxylic acid being discharged by the third step.

In an embodiment of the method for recovering a carboxylic acid according to the present disclosure, the method further includes a fifth step of distilling or stripping wastewater containing, in terms of the mass fraction, less than 0.005 of a carboxylic acid, less than 0.001 of a prepared extractant component, and 0.003 to 0.2 of an inorganic salt, the wastewater being discharged by the first step, to distill the prepared extractant component and to obtain an inorganic salt aqueous solution, and returning the distilled prepared extractant component to a primary wastewater in the first step.

### Advantageous Effects of Invention

In the present disclosure, in recovering a carboxylic acid from an aqueous solution containing a carboxylic acid, in extracting the carboxylic acid by liquid-liquid contact with the aqueous solution, a prepared extractant containing a diluent that satisfies the above-described specific conditions and a component for extracting a carboxylic acid is used, and a process including the extraction step and a subsequent distillation step is appropriately performed, such that the carboxylic acid can be efficiently recovered as a carboxylic acid anhydride with a high degree of purification.

### Brief Description of Drawings

Fig. 1 is a block diagram showing each step in an embodiment of a method for recovering a carboxylic acid according to the present disclosure.
Fig. 2 is a block diagram showing each step in another embodiment of a method for recovering a carboxylic acid according to the present disclosure.
Fig. 3 is a block diagram showing each step in still another embodiment of a method for recovering a carboxylic acid according to the present disclosure.
Fig. 4 is a block diagram showing each step in further still another embodiment of a method for recovering a carboxylic acid according to the present disclosure.

### Description of Embodiments

Hereinafter, the present disclosure will be described in more detail based on preferred embodiments.

### (Object to be treated)

In the present disclosure, a carboxylic acid is recovered from an aqueous solution containing a carboxylic acid, but the aqueous solution containing the carboxylic acid as an object to be treated is not particularly limited, and includes, but is not limited to, wastewater discharged from processes such as various chemical industries and spinning industries, a dilute carboxylic acid solution produced by microorganisms in a fermentation industry, and the like, and in particular, wastewater containing a carboxylic acid and inorganic salt(s).

Examples of the carboxylic acid targeted in the present disclosure include a carboxylic acid having 1 to 3 carbon atoms, specifically, formic acid, acetic acid, and propionic acid, and particularly, formic acid.

The inorganic salt that can be contained in the wastewater is not particularly limited, may be, for example, a metal chloride, a metal sulfate, a metal hydrogen sulfate, a metal hydroxide, a metal carbonate, a metal hydrogen carbonate, a metal phosphate, a metal hydrogen phosphate, a metal borate, and the like, and in particular, includes sodium chloride and sodium sulfate.

In addition, the wastewater may contain impurities which are components that are dissolved in a carboxylic acid and are not dissolved in water.

A carboxylic acid concentration in the aqueous solution containing a target carboxylic acid in the recovery method according to the present disclosure is in a mass fraction range of about 0.05 to 0.3, and in a case where an inorganic salt is contained, an inorganic salt concentration is in a mass fraction range of about 0.003 to 0.2.

### (First step)

The aqueous solution having a carboxylic acid concentration of 0.05 to 0.3 in terms of the mass fraction as described above is targeted, and the first step of the recovery method according to the present disclosure includes a liquid-liquid extraction step of bringing the aqueous solution into liquid-liquid contact with a prepared extractant containing a component for extracting a carboxylic acid and a diluent, the liquid-liquid contact resulting in a state that, on the aqueous solution side, the component for extracting the carboxylic acid of the prepared extractant is dissolved in less than 0.001 in term of the mass fraction and the carboxylic acid concentration in the aqueous solution is less than 0.005 in term of the mass fraction, and, on the prepared extractant side, the carboxylic acid separated with a recovery rate of not less than 90% from the aqueous solution is dissolved and water dissolved in less than 0.05 in terms of the mass fraction.

Note that in a case where the aqueous solution containing a carboxylic acid as an object to be treated further contains an inorganic salt in a mass fraction range of 0.003 to 0.2, in the liquid-liquid extraction step in the first step, the inorganic salt in the aqueous solution is dissolved in the prepared extractant in an amount of less than 0.0001 and in the water in the aqueous solution in an amount of 0.003 to less than 0.2.

Therefore, in the recovery method according to the present disclosure, in the liquid-liquid extraction step in the first step, a prepared extractant containing a component for extracting a carboxylic acid and a diluent that satisfies the following conditions is used as an extractant.

That is, the diluent used in the prepared extractant has a minimum boiling azeotrope with water, a water concentration in an azeotropic composition with the diluent is not less than 0.2, and preferably, not less than 0.5, in terms of the mass fraction, and the diluent has no azeotrope with the carboxylic acid, or
the diluent has a minimum boiling azeotrope with water, a water concentration in an azeotropic composition with the diluent is not less than 0.2, and more preferably, not less than 0.5, in terms of the mass fraction, or the diluent has a minimum boiling azeotrope with the carboxylic acid, the carboxylic acid and the diluent are separated into layers at an arbitrary ratio, and the dissolution of the diluent in the carboxylic acid is less than 0.002 in terms of the mass fraction, and
the solvent is a hydrophobic solvent typified by hydrocarbons having a boiling point higher than that of water and higher than that of carboxylic acid, and preferably a boiling point of 110 to 220°C at atmospheric pressure (for example, 1,013 ± 20 hPa).

Here, the "hydrophobic solvent" is not particularly limited, but is, for example, a solvent having a solubility in water at 25°C of less than 0.01, and more preferably, less than 0.001 in terms of the mass fraction.

Examples of such a diluent include saturated or unsaturated aliphatic and aromatic hydrocarbons as long as the above conditions are satisfied, and among them, toluene, octane, isooctane, nonane, decane, undecane, dodecane, o-xylene, m-xylene, p-xylene, ethylbenzene, and the like are preferable, and toluene, octane, decane are particularly preferable.

On the other hand, the component for extracting a carboxylic acid is a water-insoluble organic solvent that has a boiling point higher than that of the diluent, and preferably, a boiling point of 140 to 280°C in an operating pressure range of 6.67 kPa to 66.7 kPa, has a carboxylic acid distribution ratio D of "water" to "the component for extracting" (= the carboxylic acid in the component for extracting/the carboxylic acid in water) of not less than 0.3 in the present operating range, and is completely compatible with the diluent. Here, in the present specification, the "completely compatible" refers to one that becomes a uniform one liquid without being separated under the operating temperature condition of the first step.

In addition, when the carboxylic acid distribution ratio D of "water" to "the component for extracting" (= the carboxylic acid in the component for extracting/the carboxylic acid in water) is not less than 0.3 it can be determined that the extraction performance of the component to be extracted is good, and it is more preferable that the carboxylic acid distribution ratio D is not less than 0.6.

The component for extracting a carboxylic acid satisfying such conditions is not particularly limited, and examples thereof preferably include organic phosphorus compounds such as tributyl phosphate and trioctylphosphine oxide, and amide compounds such as N,N-di-n-butylformamide and N-n-butyl-N-2-ethylhexylformamide, and further preferably include tributyl phosphate and N,N-di-n-butylformamide.

In the present disclosure, when such a diluent is added to the component for extracting a carboxylic acid as the prepared extractant, it is possible to improve layer separability at the time of extraction in the first step and to suppress the movement of water to the extractant, and when water is removed in distillation in the second step described below, it has the effect of pushing water to the top of the distillation column or distillation tower with priority over the carboxylic acid, that is, it plays a role as an azeotropic agent with water, which increases the separation efficiency of the carboxylic acid. Specific examples of a combination of the component for extracting a carboxylic acid and the diluent are not particularly limited, and include tributyl phosphate and toluene, tributyl phosphate and octane, tributyl phosphate and decane, N,N-di-n-butylformamide and octane, and N,N-di-n-butylformamide and decane.

Although not particularly limited, it is desirable that a mixing ratio of the component for extracting a carboxylic acid of the prepared extractant to the diluent is, for example, a ratio of the component for extracting a carboxylic acid : the diluent = 1:5 to 9:1, preferably a ratio of the component for extracting a carboxylic acid : the diluent = 1:1 to 5:1, and more preferably a ratio of the component for extracting a carboxylic acid : the diluent = 2:1 to 3:1 in terms of mass ratio. Although not particularly limited, for example, the ratio of the component for extracting a carboxylic acid : the diluent can be a ratio of 2:1, 2.1:1, 2.2:1, 2.3:1, 2.4:1, 2.5:1, 2.55:1, 2.6:1, 2.7:1, 2.8:1, 2.9:1, or 3:1. When the mixing of the component for extracting a carboxylic acid and the diluent in the prepared extractant is extremely small as compared to the range of the mixing ratio shown here, the efficiency of the first step may be reduced and the amount of the prepared extractant required may increase. On the other hand, when the diluent is extremely small as compared to the range of the mixing ratio shown here, the azeotropic composition of water and the diluent shifts to the azeotropic composition of water and the carboxylic acid in the second step, and the yield of the carboxylic acid is significantly reduced. In addition, when the amount of the diluent is extremely small, the component for extracting a carboxylic acid has a high boiling point, and thus a diluent concentration is low in the third step, such that the effect of reducing a boiling point at the bottom of the column is small, and the temperature of the bottom of the column is high, and as a result, corrosion in the column due to the carboxylic acid and gas generation due to decomposition of the carboxylic acid may occur.

The mechanism for the liquid-liquid extraction performed in the first step is not particularly limited, and may be a static liquid-liquid contact mechanism using a filler or the like, or may be, for example, a dynamic liquid-liquid contact mechanism such as a rotating disk type countercurrent connection extractor (RDC) or a Karr column.

In addition, in the liquid-liquid extraction performed in the first step, a continuous phase may be either a carboxylic acid-containing aqueous solution (wastewater) which is an object to be treated or a prepared extractant, and a dispersed phase may also be either the prepared extractant or the carboxylic acid-containing aqueous solution (wastewater) according to the continuous phase.

Note that, in a case where the carboxylic acid-containing aqueous solution (wastewater), which is an object to be treated, contains inorganic salt(s) in addition to the carboxylic acid, when the aqueous solution is subjected to the liquid-liquid extraction in the first step and water is extracted in a mass fraction of not more than 0.05 into the prepared extractant side, the inorganic salt(s) precipitates at an interface between the aqueous phase and the extractant phase. Therefore, it is desirable that the liquid-liquid extraction device is operated at a temperature of 10 to 90°C, and more preferably at a temperature of 30 to 50°C, to increase the solubility of the inorganic salt in the aqueous phase and suppress precipitation at the interface.

In addition, in a case where the carboxylic acid-containing aqueous solution (wastewater), which is an object to be treated, contains an inorganic salt, for example, sodium sulfate or the like, in addition to the carboxylic acid, the presence of the inorganic salt improves the layer separation in the liquid-liquid extraction, and water hardly enters the extractant layer, such that a more preferable effect can be obtained. Such an action is preferably exhibited when the carboxylic acid-containing aqueous solution (wastewater), which is an object to be treated, contains an inorganic salt in a mass fraction range of 0.003 to 0.2, and more preferably, in a mass fraction range of 0.1 to 0.15, as described above. In an aspect in which the carboxylic acid-containing aqueous solution (wastewater), which is an object to be treated, does not contain an inorganic salt, it is not required to intentionally add an inorganic salt, but in some cases, it is also possible to intentionally add an inorganic salt in a predetermined amount.

In addition, as described above, in a case where the carboxylic acid-containing aqueous solution (wastewater), which is an object to be treated, contains impurities, it is desirable that, before applying the carboxylic acid-containing aqueous solution (wastewater) to the first step, a treatment of removing the impurities by filtering the wastewater is performed, or the carboxylic acid is recovered in the prepared extractant in the first step, such that the impurities that precipitate at the interface with the extractant phase are extracted from a nozzle provided at the top or the bottom of the liquid-liquid extraction device together with the prepared extractant and the wastewater, and the impurities are removed by separating a liquid containing the extracted impurities into impurities and a liquid by centrifugation and mixing a recovered liquid with the primary wastewater in the first step. These treatments may be used alone or in combination.

### (Second step)

The second step of the recovery method according to the present disclosure includes a step of distilling the prepared extractant containing a carboxylic acid and water that is subjected to the first step, azeotropically distilling the diluent in the prepared extractant and water, separating the distillate into an extractant component layer mainly containing a diluent and an aqueous layer with a decanter provided in a distillation column and then discharging the aqueous layer, mixing the discharged water with a primary aqueous solution or a secondary aqueous solution in the first step or disposing of the discharged water as wastewater while returning the extractant component layer mainly containing a diluent to the distillation step as reflux, and discharging the prepared extractant from which water is removed through the bottom of the column. Note that, in the present specification, the form of the "decanter" is not particularly limited as long as the diluent layer and the aqueous layer can be separated from each other, and for example, the decanter is not limited to one that performs separation by using an inclination.

Note that, the "extractant component layer mainly containing a diluent" in the decanter provided in the distillation column may strictly contain trace amounts of a carboxylic acid and a component for extracting a carboxylic acid in addition to the diluent component. However, since the extractant component layer is returned to the distillation step as reflux, there is no particular problem even when the carboxylic acid and the component for extracting a carboxylic acid are contained.

In the present disclosure, since the diluent blended in the prepared extractant as described above has a minimum boiling azeotrope with water, a water concentration in the azeotropic composition is not less than 0.2 in terms of the mass fraction, and the diluent has no azeotrope with the carboxylic acid; or the diluent has a minimum boiling azeotrope with water, a water concentration in the azeotropic composition with the diluent is not less than 0.2 in terms of the mass fraction, the carboxylic acid has a minimum boiling azeotrope with the diluent, and the component for extracting a carboxylic acid in the prepared extractant has a boiling point higher than that of the diluent and has a high affinity with the carboxylic acid, when the prepared extractant containing the carboxylic acid and water that is subjected to the first step is distilled, the extractant effect in distillation is in play, and water can be preferentially distilled by suppressing vaporization of the carboxylic acid.

Note that, the aqueous layer that is distilled in this way and is separated from the extractant component layer mainly containing a diluent with the decanter provided in the distillation column may also strictly contain a trace amount of a carboxylic acid. However, since the separated water can be mixed with the primary or secondary aqueous solution in the first step and can be subjected to each step again, even when the carboxylic acid is contained in the aqueous layer, there is no particular problem.

The distillation in the second step is not particularly limited, but for example, it is desirable that the distillation is performed at a degree of reduced pressure of 6.67 to 66.7 kPa, and more preferably at a degree of reduced pressure of 13.3 to 26.7 kPa. By performing the distillation at a reduced pressure in the second step, the bottom temperature is lowered, and decomposition of the carboxylic acid and corrosion in the column can be suppressed.

The distillation conditions are not particularly limited, but for example, the number of trays in a plate column is set to about 5 to 10 and a reflux ratio is set to about 1 to 3.

In addition, when a compositional balance in the column is lost, a prepared extractant, and more preferably, a recycled prepared extractant in a third step to be described later, is supplied from an arbitrary tray above a raw material supply tray, such that an effect of removing formic acid at the bottom of the column by an extraction effect of the component for extracting a carboxylic acid is obtained, and thus, the loss of formic acid can be further reduced.

Note that, in a case where the carboxylic acid-containing aqueous solution (wastewater), which is an object to be treated, contains inorganic salt(s) in addition to the carboxylic acid, there is a possibility that the inorganic salt(s) dissolved in water precipitates at the bottom of the distillation column when water is distilled away from the prepared extractant containing the carboxylic acid and water in this second step. In order to remove this, it is desirable to provide a filtering step in a line through which a bottom liquid is transferred to the third step described below.

### (Third step)

The third step of the recovery method according to the present disclosure is characterized in that the prepared extractant containing the carboxylic acid discharged in the second step is distilled again, and
in a case that there is no azeotrope between the carboxylic acid and the diluent in the prepared extractant, a purified carboxylic acid containing, in terms of the mass fraction, less than 0.01 of water and less than 0.01 of the prepared extractant is discharged through the top of the column, and the prepared extractant from which the carboxylic acid and water are removed, the prepared extractant being discharged through the bottom of the column, is returned to the first step, or

in a case that the diluent and the carboxylic acid in the prepared extractant have a minimum boiling azeotrope, the diluent and the carboxylic acid distilled off by azeotropic distillation are separated into an extractant component layer mainly containing a diluent and a carboxylic acid layer with a decanter provided in a distillation column, the carboxylic acid layer containing, in terms of the mass fraction, less than 0.01 of water and less than 0.01 of the prepared extractant is discharged to obtain a purified carboxylic acid, the extractant component layer mainly containing a diluent is returned to the distillation step as reflux, and the prepared extractant from which the carboxylic acid and water are removed, the prepared extractant being discharged through the bottom of the column, is returned to the first step.

In the recovery method of the present disclosure, in addition to the water removal from the prepared extractant by distillation in the second step, the distillation is carried out again in the third step. Thus, the carboxylic acid from the prepared extractant can be obtained as a purified carboxylic acid. Note that the presence of the diluent at the bottom of the distillation column during the distillation suppresses an increase in bottom temperature of the column, and thus there is also an effect that the heat resistance requirement of the material constituting the apparatus is relaxed and the choices of materials are widened.

The distillation in the third step is not particularly limited, but for example, it is desirable that the distillation is performed at a degree of reduced pressure of 6.67 to 66.7 kPa, and more preferably at a degree of reduced pressure of 13.3 to 26.7 kPa. Since the distillation is performed at a reduced pressure in the third step to reduce an azeotropic point of the carboxylic acid with the diluent, corrosion in the column and decomposition of the carboxylic acid can be suppressed, and the prepared extractant recycled in the third step is reused in the first step, such that the possibility of decomposition due to a thermal history and generation of impurities can be suppressed.

The distillation conditions are not particularly limited, but for example, the number of trays in a plate column is set to about 5 to 10 and a reflux ratio is set to about 3 to 5.

In addition, in order to improve the recovery rate of the carboxylic acid, the extractant component layer mainly containing a diluent obtained from the decanter in the third step is supplied to the column from an arbitrary tray below the raw material supply tray separately from the reflux, such that it is possible to promote the azeotrope of the diluent with the carboxylic acid concerning the carboxylic acid suppressed by the extraction effect of the component for extracting a carboxylic acid.

### (Fourth step)

In an embodiment of the method for recovering a carboxylic acid according to the present disclosure, the method can further include a fourth step of obtaining a carboxylic acid anhydride containing not less than 0.99 and more preferably, not less than 0.995 of a carboxylic acid, and not more than 0.002 of water by re-distilling the carboxylic acid discharged by the third step, in the state containing, in terms of the mass fraction, less than 0.01 of water and less than 0.01 of the prepared extractant, and thereby distilling water and the prepared extractant component off.

If necessary, the degree of purification of the carboxylic acid can be increased by re-distilling the carboxylic acid obtained in the third step. Note that the number of distillations in the fourth step can be not only one but also more than one.

The distillation in the fourth step, which is performed if necessary, is not particularly limited, but for example, it is desirable that the distillation is performed at a degree of reduced pressure of 6.67 to 66.7 kPa, and more preferably at a degree of reduced pressure of 26.7 to 40.0 kPa.

The distillation conditions are not particularly limited, and examples thereof include a case where a product is extracted through the top of the column having the number of trays of 5 to 10 and a reflux ratio of about 3 to 5 in the plate column, and a case where a product is extracted from the middle of the column in a side cut in a total reflux state. Therefore, water or the component for extracting a carboxylic acid is concentrated at the bottom of the column, and the diluent component is concentrated at the top of the column, such that a high-purity carboxylic acid can be recovered. Although not particularly limited, for example, formic anhydride containing formic acid in a mass fraction of 0.998 and water in a mass fraction of 0.002 can be obtained.

### (Fifth step)

In an embodiment of the method for recovering a carboxylic acid according to the present disclosure, the method can further include, if necessary, a fifth step of distilling or stripping wastewater containing, in terms of the mass fraction, less than 0.005 of a carboxylic acid, less than 0.001 of the component for extracting the carboxylic acid of the prepared extractant, and 0.003 to 0.2 of an inorganic salt, the wastewater being discharged by the first step, to distill the prepared extractant component and the carboxylic acid away and to obtain an inorganic salt aqueous solution, and returning the distilled prepared extractant component and the carboxylic acid to a primary wastewater in the first step.

In an aspect in which the carboxylic acid-containing aqueous solution (wastewater) as an object to be treated further contains inorganic salt(s), for example, in a case where any production or treatment process in which the wastewater results uses the inorganic salt(s) in the process, such a fifth step is provided, in addition to recovery of the carboxylic acid, the inorganic salt can be recovered as an inorganic salt aqueous solution, and reuse of raw material resources is further promoted.

The distillation conditions in the fifth step performed if necessary are not particularly limited, and for example, distillation at atmospheric pressure of about 1,013 ± 20 hPa can be performed.

The distillation conditions are not particularly limited, and examples thereof include a case where the number of trays in a plate column is 5 to 10, and a product is extracted as an inorganic salt aqueous solution through the bottom of the column in a total reflux state.

### Examples

Hereinafter, the present disclosure will be described more specifically based on Examples.

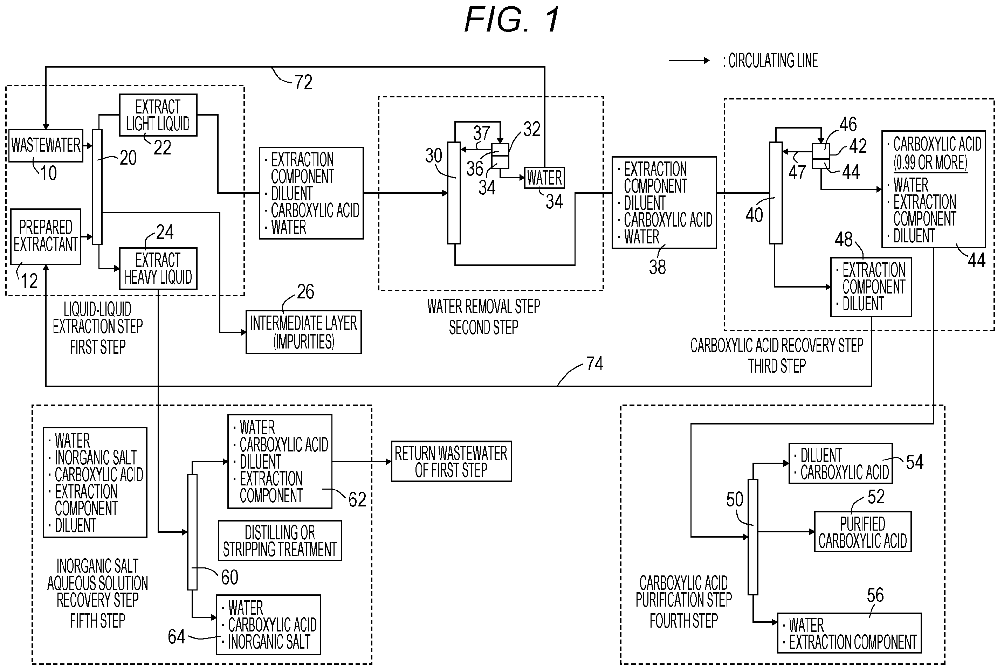

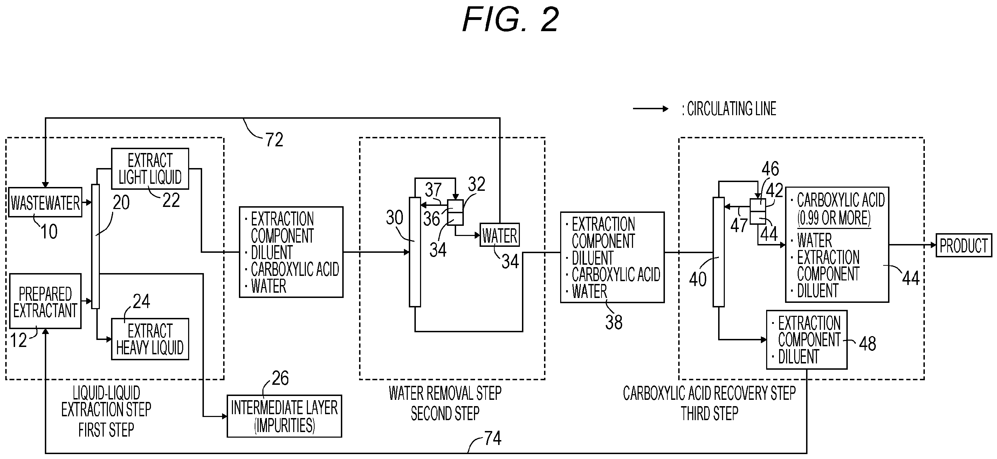

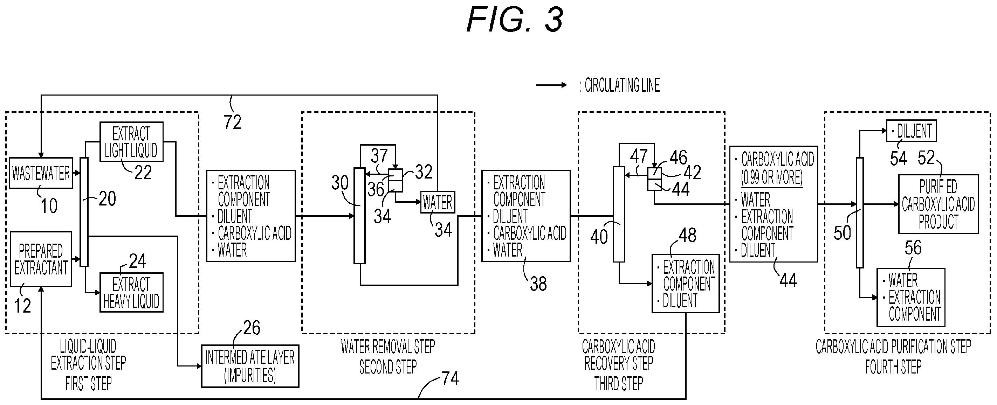

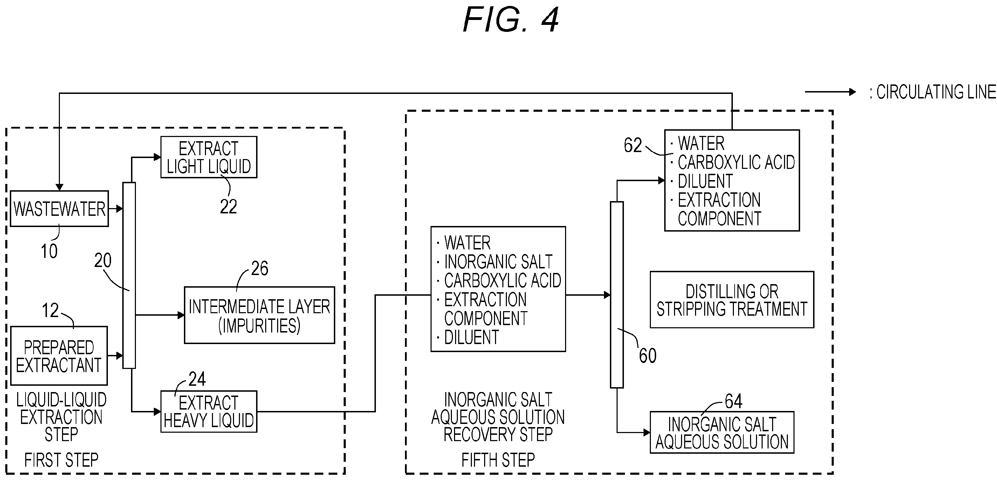

Figs. 1 to 4 are block diagrams showing the respective steps in an embodiment of a method for recovering a carboxylic acid according to the present disclosure. Note that Fig. 1 is a block diagram showing a general aspect including all of the first to fifth steps in an embodiment of the method for recovering a carboxylic acid according to the present disclosure, Fig. 2 is a block diagram showing an aspect including the first to third steps in an embodiment of the method for recovering a carboxylic acid according to the present disclosure, Fig. 3 is a block diagram showing an aspect including the first to fourth steps in an embodiment of the method for recovering a carboxylic acid according to the present disclosure, and Fig. 4 is a block diagram showing an aspect including the first to fifth steps in an embodiment of the method for recovering a carboxylic acid according to the present disclosure.

Note that, in the following Examples, each component was analyzed according to the following method.

### (Water)

Water was quantitatively determined by gas chromatography under the following conditions.
Instrument: GC-2014 (manufactured by Shimadzu Corporation) Detector: Thermal conductivity detector
Column: Chromosorb 101 (inner diameter: 2 mm, length: 1.83 m) Column temperature rise condition: 90°C → 10°C/min temperature rise → 220°C (hold for 47 minutes)
Inlet condition: 250°C, carrier gas flow rate: 4 ml/min

### (Carboxylic acid)

When a carboxylic acid concentration was 0.5 or less in terms of mass fraction, the carboxylic acid concentration was determined by neutralization titration using an automatic potentiometric titrator under the following conditions. When the carboxylic acid concentration was not less than 0.5 in terms of mass fraction, it was calculated by subtracting each component from 100.
Instrument: AT-710 (manufactured by Kyoto Electronics Manufacturing Co., Ltd.)
Titration reagent: 0.1 mol/L ethanolic potassium hydroxide aqueous solution (manufactured by FUJIFILM Wako Pure Chemical Corporation)

### (Other volatile components)

Other volatile components were quantitatively determined by gas chromatography under the following conditions.
Instrument: GC-2014 (manufactured by Shimadzu Corporation) Detector: Hydrogen flame ion detector
Column: G-100 (inner diameter: 1.2 mm, length: 40 m, film thickness: 3.0 µm)
Column temperature rise condition: 80°C → 10°C/min temperature rise → 240°C (hold for 44 minutes)
Inlet condition: 250°C, carrier gas flow rate: 15 ml/min

### (Nonvolatile component)

The weight of the nonvolatile component was determined by weighing about 5.0 g of the liquid on a φ100 ml Petri dish, and measuring the weight after being allowed to stand for 24 hours at a reduced pressure of about 1.3 kPa in a vacuum dryer set at 140°C.

### Example 1

As a carboxylic acid-containing aqueous solution to be treated, wastewater containing formic acid in a mass fraction of 0.2, sodium sulfate in a mass fraction of 0.14, impurities in a mass fraction of 0.0005, and the balance water was prepared. The specific gravity of the wastewater was 1.18 at 20°C.

### (First step)

As illustrated in Figs. 1 and 2, wastewater 10 was introduced into a filling type extraction column liquid-liquid extraction device 20 filled with a regular filler and having a column diameter of 65 mm and a height equivalent to the number of theoretical trays of 3, a prepared extractant 12 as a mixture of tributyl phosphate (TBP) as a component for extracting a carboxylic acid and decane as a diluent at a mass ratio (TBP : decane) of 3:1, which was introduced oppositely into the liquid-liquid extraction device 20 was heated at 40°C, and the wastewater 10, that is, 320 kg of the wastewater as a wastewater treatment amount, was brought into liquid-liquid contact with 1,040 kg of the prepared extractant 12 at a wastewater supply flow rate of 40 kg/h and a prepared extractant supply flow rate of 130 kg/h under a treatment condition of 8 hours. The specific gravity of the prepared extractant was 0.89 at 20°C. Note that the prepared extractant 12 was used as a continuous phase and the wastewater 10 was used as a dispersed phase.

An extractant layer (light liquid) 22 which extracted formic acid therein by the liquid-liquid extraction device was led off from a nozzle provided at the top of the liquid-liquid extraction device 20, and an aqueous layer (heavy liquid) 24 from which formic acid was removed was led off from a nozzle provided at the other bottom. Further, impurities precipitated at an interface (intermediate layer 26) with the aqueous layer by this liquid-liquid extraction operation were removed together with the aqueous layer 24 from the nozzle provided at the bottom of the liquid-liquid extraction device, and a liquid containing the extracted impurities was separated into the impurities and the liquid by centrifugation to remove the impurities 26.

As a result of examining the composition of 1,125 kg of the extractant layer (light liquid) 22 extracted by the first step, it was found that formic acid was 0.056 in terms of the mass fraction, decane was 0.231 in terms of the mass fraction, water was 0.020 in terms of mass fraction, sodium sulfate was 0.00001 in terms of the mass fraction, and the balance was TBP.

### (Second step)

The extractant layer (light liquid) 22 was then charged into a distillation tower (continuous plate column) 30 having 8 actual trays at a supply rate of 200 kg/h from the top of the column with the fourth tray as a raw material supply tray, and distillation was performed under conditions of a column bottom temperature of 118 to 120°C and a column top temperature of 52 to 53°C at a reduced pressure of 13.3 kPa. Therefore, decane and water were azeotropically distilled from the top of the column, and after being separated into an extractant component layer mainly containing a diluent and an aqueous layer by a decanter 32 provided in the distillation tower 30, the aqueous layer was discharged. The discharged water 34 was mixed with the primary aqueous solution (wastewater 10) in the first step through a return line 72. On the other hand, the extractant component layer 36 mainly containing a diluent was returned to the distillation tower 30 as reflux through a reflux line 37 at a reflux ratio of 2. Then, a prepared extractant 38 from which water was removed was led out of the bottom of distillation tower 30 at 180 kg/h.

As a result of examining the composition of 1,085 kg of the prepared extractant 38 from which water was removed, the prepared extractant 38 being extracted by the second step, it was found that formic acid was 0.055 in terms of the mass fraction, decane was 0.236 in terms of the mass fraction, water was less than 0.0003 in terms of the mass fraction, and the balance was TBP.

Note that a filter (not illustrated) including a bag filter having a filtration accuracy of 0.5 µm was provided on a line 39 through which the prepared extractant 38 from which the water was removed was transferred to a third step to be described below, the prepared extractant 38 being extracted by the second step. Water was distilled from the prepared extractant containing formic acid and water in the second step, such that there was a possibility that sulfate dissolved in water precipitated at the bottom of the distillation tower, but no precipitate was observed at the bottom of the distillation tower through the operation for about 5 hours due to the installation of the filter.

### (Third step)

The prepared extractant 38 from which water was removed, the prepared extractant 38 being extracted by the second step, was then charged into a distillation tower (continuous filling column) 40 having 8 theoretical trays at a supply rate of 180 kg/h using the fourth tray from the top of the column, as a raw material supply tray, and a distillation operation was performed for about 6 hours under conditions of a reduced pressure of 11.3 kPa, a bottom temperature of 130 to 131°C, and a top temperature of 85 to 90°C. Therefore, the diluent (decane) and the carboxylic acid (formic acid) in the prepared extractant were distilled by azeotropic distillation and were separated into an extractant component layer 46 mainly containing a diluent and a formic acid layer 44 with a decanter 42 provided in the distillation tower 40, and then the formic acid layer 44 was discharged to obtain purified formic acid. On the other hand, the extractant component layer 46 mainly containing a diluent was returned to the distillation tower 40 as reflux through a reflux line 47 at a reflux ratio of 4. In addition, the prepared extractant 48 from which formic acid and water were removed, the prepared extractant 48 being discharged at 150 kg/h from the bottom of the distillation tower 40, was returned to the first step through a return line 74, and the composition was adjusted, if necessary, and reused together with the prepared extractant 12.

As a result of examining the composition of 60 kg of the formic acid layer 44 obtained as the purified formic acid by the third step, it was found that formic acid was contained in a mass fraction of 0.99, water was 0.003 in terms of the mass fraction, TBP was 0.004 in terms of the mass fraction, decane was 0.003 in terms of the mass fraction, and a degree of purification was high.

In addition, as a result of examining the composition of the prepared extractant 48 from which formic acid and water were removed, the prepared extractant 48 being discharged through the bottom of the column in the third step, it was found that formic acid was 0.0002 in terms of the mass fraction, decane was 0.251 in terms of the mass fraction, water was 0.0002 in terms of the mass fraction, and the balance was TBP.

### Example 2

### (Fourth step)

As illustrated in Figs. 1 and 3, the formic acid layer 44 obtained in Example 1 was charged into a distillation tower (continuous filling column) 50 having 7 theoretical trays at a supply rate of 45 kg/h, distillation was performed again under conditions of a reduced pressure of 30 kPa, a bottom temperature of 68 to 70°C, and a top temperature of 63 to 64°C, and water and a prepared extractant component 56 were removed from the middle of the column in a total reflux state. As a result, as the resulting purified carboxylic acid product 52, formic anhydride containing formic acid in a mass fraction of 0.998 and water in a mass fraction of 0.002 was obtained.

### Example 3

### (Fifth step)

In Example 1, as a result of examining the composition of 235 kg of the aqueous layer (heavy liquid) 24 extracted from the nozzle provided at the bottom of the liquid-liquid extraction device 20 in the first step, it was found that sodium sulfate was 0.19 in terms of the mass fraction, formic acid was 0.002 in terms of the mass fraction, TBP was 0.0008 in terms of the mass fraction, decane was less than 0.00003 in terms of the mass fraction, and the balance was water. As illustrated in Figs. 1 and 4, the aqueous layer 24 was charged into a distillation tower (continuous plate column) 60 having 6 actual trays at 60 kg/h using the third tray from the top of the column, as a raw material supply tray, and distillation was performed at 101.3 kPa, a column bottom temperature of 102 to 103°C, and a column top temperature of 100°C under a total reflux condition.

When the composition of a bottom product 64 extracted from the bottom of the distillation tower (continuous plate column) 60 at 55 kg/h was examined, it was found that sodium sulfate was 0.191 in terms of the mass fraction, formic acid was 0.002 in terms of the mass fraction, and the balance was water and was reused as a sodium sulfate aqueous solution for industrial use.

On the other hand, when the composition of a distillate 62 removed by distillation from the top of the distillation tower (continuous plate column) 60 was examined, it was found that formic acid was 0.0002 in terms of the mass fraction, TBP was 0.002 in terms of the mass fraction, decane was less than 0.0001 in terms of the mass fraction, and the balance was water. Therefore, it was possible to return the distillate 62 to the primary aqueous solution (wastewater 10) in the first step.

### Example 4

As a carboxylic acid-containing aqueous solution to be treated, a simulated wastewater containing formic acid in a mass fraction of 0.2 and the balance water was prepared, whereas other simulated wastewater containing formic acid in a constant mass fraction of 0.2, sodium sulfate in mass fractions of 0.01, 0.1, and 0.2, and the balance water were prepared.

The wastewater was transferred to a separatory funnel, and the prepared extractant 12 as a mixture of TBP and decane having a mass ratio (TBP : decane) of 2:1 was used at a ratio of 1 concerning 1 of the wastewater in terms of the mass ratio, and single extruction was performed by sufficient shaking at (23) °C.

After standing for 5 minutes, an aqueous layer (heavy liquid) in the separatory funnel was discharged, and an extractant layer (light liquid) was extracted. The composition of the water and the layer separability in the extracted extractant layer (light liquid) were examined, and the results were obtained as shown in Table 1.

**[Table 1]**

| (Composition is based on the mass fraction) | | |
|---|---|---|
| Sodium sulfate composition in simulated wastewater | Water composition | Layer separability |
| 0 | 0.029 | △ |
| 0.01 | 0.029 | △ |
| 0.1 | 0.027 | ○ |
| 0.2 | 0.024 | ○ |

| | | |
|---|---|---|
| * The layer separability was determined by allowing the mixture to stand after mixing and measuring the time taken for the mixture to become uniform two liquids without interfacial turbidity, and the case where the time was within 30 seconds was marked as ○, the case where the time was within 2 minutes was marked as △, and the case where the time was longer than 2 minutes was marked as ×. | | |

As shown in Table 1, the presence of the inorganic salt in the wastewater exhibited the effect of suppressing the movement of water (reducing the load in the second step) and improving the layer separability.

### Examples 5 to 13

In the carboxylic acid-containing aqueous solution to be treated, a simulated wastewater containing formic acid in a mass fraction of 0.18, sodium sulfate in a mass fraction of 0.15, impurities in a mass fraction of 0.0005, and the balance water was used, this wastewater was transferred to a separatory funnel, the prepared extractant shown in the composition of Table 2 was used at a ratio of 1 concerning 1 of the wastewater in terms of the mass ratio, and single extraction was performed through by sufficient shaking.

After standing, an aqueous layer (heavy liquid) in the separatory funnel was discharged, and an extractant layer (light liquid) was extracted.

The composition of the extracted extractant layer (light liquid) was examined, and the formic acid composition, water composition, and distribution ratio of formic acid in the extraction liquid from which formic acid was extracted were as shown in Table 3.

**[Table 2]**

| (Blending ratio is based on the weight ratio) | | | |
|---|---|---|---|
| Example | Extractant type | Diluent type | Blending ratio (extractant : diluent) |
| Example 5 | Trioctyl amine | None | - |
| Example 6 | Trioctyl amine | Toluene | 1:1 |
| Example 7 | Trioctyl amine | Octane | 2:1 |
| Example 8 | Dibutylformamide | None | - |
| Example 9 | Dibutylformamide | Octane | 1:1 |
| Example 10 | Dibutylformamide | Decane | 1:1 |
| Example 11 | Tributyl phosphate | None | - |
| Example 12 | Tributyl phosphate | Octane | 3:1 |
| Example 13 | Tributyl phosphate | Decane | 3:1 |

**[Table 3]**

| (Composition is based on the mass fraction, and blending ratio is formic acid in extraction component/formic acid in water.) | | | | |
|---|---|---|---|---|
| Example | Formic acid composition | Water composition | Distribution ratio | Layer separability |
| Example 5 | 0.14 | 0.06 | 21.65 | × |
| Example 6 | 0.13 | 0.02 | 2.52 | × |
| Example 7 | 0.14 | 0.02 | 3.81 | △ |
| Example 8 | 0.12 | 0.08 | 1.74 | △ |
| Example 9 | 0.08 | 0.02 | 0.59 | ○ |
| Example 10 | 0.08 | 0.02 | 0.58 | ○ |
| Example 11 | 0.10 | 0.09 | 0.91 | △ |
| Example 12 | 0.08 | 0.03 | 0.61 | △ |
| Example 13 | 0.08 | 0.03 | 0.62 | ○ |

| | | | | |
|---|---|---|---|---|
| * The layer separability was determined by allowing the mixture to stand after mixing and measuring the time taken for the mixture to become uniform two liquids without interfacial turbidity, and the case where the time was within 30 seconds was marked as ○, the case where the time was within 2 minutes was marked as △, and the case where the time was longer than 2 minutes was marked as ×. | | | | |

Distillation steps were performed on a lab scale for Examples 7, 9, 10, 11, and 13.

The distillation was performed using a glass Oldershaw having 30 theoretical trays for all the devices used and a mantle heater as a heating source at a reflux ratio of 5 under a reduced pressure condition.

As a result, the water composition and the formic acid composition at the top of the column and the water composition and the formic acid composition at the bottom of the column were obtained as shown in Tables 4 and 5, respectively.

**[Table 4]**

| (Composition is based on the mass fraction.) | | | | | |
|---|---|---|---|---|---|
| Example | Operating pressure | Formic acid composition | Water composition | Diluent composition | Water dissolubility |
| Example 7 | 10.7 kPa | 0.48 | 0.06 | 0.46 | × |
| Example 9 | 13.3 kPa | 0.02 | 0.3 | 0.65 | △ |
| Example 10 | 19.9 kPa | 0.03 | 0.68 | 0.26 | ○ |
| Example 11 | 13.3 kPa | 0.01 | 0.99 | - | ○(*) |
| Example 13 | 13.3 kPa | 0.08 | 0.78 | 0.14 | ○ |

| | | | | | |
|---|---|---|---|---|---|
| * The water dissolubility was marked as ○ when water was preferentially distilled from the initial distillation (not less than 0.5 in terms of the mass fraction), the case where the water dissolubility was not less than 0.25 was marked as △, and the case where the water dissolubility was less than 0.25 was marked as ×. | | | | | |

**[Table 5]**

| (Composition is based on the mass fraction.) | | | | | |
|---|---|---|---|---|---|
| Example | Operating pressure | Formic acid composition at bottom of column | Water composition at bottom of column | Formic acid dissolubility | Prepared extractant form |
| Example 7 | 10.7 kPa | 0.004 | 0.0005 | △ | × (*) |
| Example 9 | 13.3 kPa | 0.03 | 0.001 | × | ○ |
| Example 10 | 19.9 kPa | 0.004 | 0.001 | △ | ○ |
| Example 11 | 13.3 kPa | 0.2 (*) | 0.004 (*) | × (*) | ○ |
| Example 13 | 13.3 kPa | 0.0002 | 0.0007 | ○ | ○ |

| | | | | | |
|---|---|---|---|---|---|
| * Since the recycled prepared extractant was recirculated and reused, the formic acid dissolubility was marked as ○ when the formic acid concentration in the prepared extractant at the bottom of the column was 0.001 or less in terms of the mass fraction, △ when the formic acid concentration in the prepared extractant at the bottom of the column was 0.01 or less in terms of the mass fraction, and × when the formic acid concentration in the prepared extractant at the bottom of the column was more than 0.01 in terms of the mass fraction. | | | | | |

The case where the prepared extractant form was not layer-separated or precipitated was marked as ○, the case where the prepared extractant form was layer-separated was marked as △, and the case where the prepared extractant form was layer-separated and precipitated was marked as ×.

Regarding a formic acid solution in which water obtained in Example 13 was 0.04 in terms of the mass fraction, TBP was 0.0001 in terms of the mass fraction, decane was 0.001 in terms of the mass fraction, and the balance was formic acid, purified formic acid recovery was performed by side-cutting in a total reflux state using a glass Oldershaw having 10 theoretical trays under a reduced pressure condition of 26.5 kPa using a mantle heater as a heating source.

As a result, when the composition of the side-cut fraction obtained from the middle of the column was examined, it was possible to obtain formic anhydride containing formic acid in a mass fraction of 0.998 and water in a mass fraction of 0.002.

As the inorganic salt-containing aqueous solution to be treated in the fifth step, a simulated wastewater containing formic acid in a mass fraction of 0.004, TBP in a mass fraction of 0.0008, sodium sulfate in a mass fraction of 0.15, and the balance water was subjected to a distillation test in a laboratory scale in a total reflux state at 1,013 kPa using a glass Oldershaw having 5 theoretical trays and a mantle heater as a heating source.

As a result, when the composition of the column bottom liquid was examined, an inorganic salt aqueous solution containing, by mass fraction, 0.004 of formic acid and 0.18 of sodium sulfate and containing the balance water was obtained.

### Reference Signs List

- 10: Wastewater
- 12: Prepared extractant
- 20: Liquid-liquid extraction device
- 22: Extractant layer (light liquid)
- 24: Aqueous layer (heavy liquid)
- 26: Intermediate layer
- 30, 40, 50, 60: Distillation tower
- 32, 42: Decanter
- 34: Discharged water
- 36: Extractant component layer mainly containing diluent
- 37: Reflux line
- 38: Prepared extractant from which water is removed
- 44: Formic acid layer
- 46: Extractant component layer mainly containing diluent
- 48: Prepared extractant from which formic acid and water are removed
- 52: Purified carboxylic acid product
- 62: Distillate
- 64: Bottom product
- 72, 74: Return line

## Claims

1. A method for recovering a carboxylic acid, with a proviso that the carboxylic acid is separated from an aqueous solution containing water and the carboxylic acid and having a carboxylic acid concentration of 0.05 to 0.3 in terms of mass fraction and the separated carboxylic acid is purified and obtained as a carboxylic acid anhydride containing less than 0.01 of water and not less than 0.99 of carboxylic acid in terms of mass fraction, the method comprising:
a) a first step comprising a liquid-liquid extraction step of bringing the aqueous solution into liquid-liquid contact with a prepared extractant, the prepared extractant containing a component for extracting the carboxylic acid and a diluent to dissolve the component, the liquid-liquid contact resulting in a state that, on the aqueous solution side, the component of the prepared extractant is dissolved in less than 0.001 in term of mass fraction and the carboxylic acid concentration in the aqueous solution is less than 0.005 in term of mass fraction, and, on the prepared extractant side, the carboxylic acid separated with a recovery rate of not less than 90% from the aqueous solution is dissolved and water dissolved in less than 0.05 in terms of mass fraction;
b) a second step comprising a step of distilling the prepared extractant containing the carboxylic acid and water that is subjected to the first step, azeotropically distilling the diluent in the prepared extractant and water, separating the distillate into an extractant component layer mainly containing the diluent and an aqueous layer with a decanter provided in a distillation column and then discharging the aqueous layer, mixing the discharged water with a primary aqueous solution or a secondary aqueous solution in the first step or disposing of the discharged water as wastewater while returning the extractant component layer mainly containing the diluent to the distillation step as reflux, and discharging the prepared extractant containing the carboxylic acid through a bottom of the column; and
c) a third step comprising re-distilling the prepared extractant containing the carboxylic acid discharged by the second step, and
in a case where there is no azeotrope between the carboxylic acid and the diluent in the prepared extractant, discharging, through a top of the column, a purified carboxylic acid containing, in terms of mass fraction, less than 0.01 of water and less than 0.01 of the prepared extractant, and returning, to the first step, the prepared extractant from which the carboxylic acid and water are removed, the prepared extractant being discharged through the bottom of the column, or
in a case where the carboxylic acid and the diluent in the prepared extractant have a minimum boiling azeotrope, distilling the diluent and the carboxylic acid by azeotropic distillation, separating the distillate into an extractant component layer mainly containing the diluent and a carboxylic acid layer with a decanter provided in a distillation column, discharging the carboxylic acid layer containing, in terms of mass fraction, less than 0.01 of water and less than 0.01 of the prepared extractant to obtain the purified carboxylic acid while returning the extractant component layer mainly containing the diluent to the distillation step as reflux, and returning, to the first step, the prepared extractant from which the carboxylic acid and water are removed, the prepared extractant being discharged through a bottom of the column,
the diluent in the prepared extractant being a hydrophobic solvent having a boiling point higher than that of water and higher than that of the carboxylic acid at atmospheric pressure, which is **characterized in that** the diluent has a minimum boiling azeotrope with water, a water concentration in an azeotropic composition with the diluent is not less than 0.2 in terms of the mass fraction, and the diluent has no azeotrope with the carboxylic acid, or
the diluent in the prepared extractant being a hydrophobic solvent having a boiling point higher than that of water and higher than that of the carboxylic acid at atmospheric pressure, which is **characterized in that** the diluent has a minimum boiling azeotrope with water, a water concentration in an azeotropic composition with the diluent is not less than 0.2 in terms of mass fraction, the carboxylic acid has a minimum boiling azeotrope with the diluent, the carboxylic acid and the diluent are separated into layers at any ratio, and the dissolution of the diluent in the carboxylic acid is less than 0.002 in terms of mass fraction, and
the component for extracting the carboxylic acid being a poorly water-soluble organic solvent that has a boiling point higher than that of the diluent, has a carboxylic acid distribution ratio D of "water" to "the component for extracting" (= the carboxylic acid in the component for extracting/the carboxylic acid in water) of not less than 0.3, and is completely compatible with the diluent.

2. The method according to claim 1, wherein the aqueous solution further contains an inorganic salt in a mass fraction range of 0.003 to 0.2, and in the liquid-liquid extraction step in the first step, the inorganic salt in the aqueous solution is dissolved in the prepared extractant in an amount of less than 0.0001 and in the water in the aqueous solution in an amount of 0.003 to less than 0.2.

3. The method according to claim 1 or 2, wherein the carboxylic acid contained in the aqueous solution is selected from the group consisting of formic acid, acetic acid, and propionic acid.

4. The method according to any one of claims 1 to 3, wherein the inorganic salt contained in the aqueous solution is selected from the group consisting of a metal chloride, a metal sulfate, a metal hydrogen sulfate, a metal hydroxide, a metal carbonate, a metal hydrogen carbonate, a metal phosphate, a metal hydrogen phosphate, and a metal borate.

5. The method according to any one of claims 1 to 4, wherein a boiling point of the diluent in the prepared extractant is 110 to 220°C at atmospheric pressure, and is a hydrophobic solvent having a solubility in water of less than 0.001 in terms of mass fraction at 25°C, and wherein the diluent is preferably at least one selected from the group consisting of toluene, octane, isooctane, nonane, decane, undecane, dodecane, o-xylene, m-xylene, p-xylene, and ethylbenzene.

6. The method according to any one of claims 1 to 5,
wherein the aqueous solution contains impurities that are dissolved in the carboxylic acid and are not dissolved in water, and
before applying the aqueous solution to the first step, a step of removing the impurities by filtering the aqueous solution is performed, or the carboxylic acid is recovered in the prepared extractant in the first step, such that the impurities that precipitate at an interface with an extractant phase are extracted from a nozzle provided at a top or a bottom of a liquid-liquid extraction device together with the prepared extractant and the aqueous solution, and the impurities are removed by separating a liquid containing the extracted impurities into impurities and a liquid by filtration or centrifugation and mixing a recovered liquid with the primary aqueous solution in the first step.

7. The method according to any one of claims 1 to 6, wherein the component for extracting the carboxylic acid in the prepared extractant is selected from the group consisting of an organophosphorus compound and an amide compound.

8. The method according to any one of claims 1 to 7, wherein a mixing ratio of the component for extracting the carboxylic acid to the diluent in the prepared extractant is a ratio of the component for extracting the carboxylic acid : the diluent of 1:5 to 9:1 in terms of mass ratio.

9. The method according to any one of claims 2 to 8, wherein when the aqueous solution containing the inorganic salt is subjected to the first step, a liquid-liquid extraction device is operated at a temperature of 10 to 90°C in a state where the inorganic salt is dissolved.

10. The method according to any one of claims 1 to 9, wherein the second step and/or the third step is performed at a degree of reduced pressure of 6.67 to 66.7 kPa.

11. The method according to any one of claims 2 to 10, wherein a filtering step is added to a line through which a bottom liquid is transferred in the third step to remove the inorganic salt that precipitates at the bottom of the distillation column by distilling water from the prepared extractant containing the carboxylic acid and water in the second step.

12. The method according to any one of claims 1 to 11, wherein the second step includes a line through which the prepared extractant is supplied to the column from an arbitrary tray above a raw material supply tray, separately from the reflux of the extractant component layer mainly containing the diluent, to improve a recovery rate of the carboxylic acid.

13. The method according to any one of claims 1 to 12, wherein the third step includes a line through which the extractant component layer mainly containing a diluent is supplied to the column from an arbitrary tray below a raw material supply tray, separately from the reflux of the extractant component layer mainly containing the diluent, to improve a recovery rate of the carboxylic acid.

14. The method according to any one of claims 1 to 13, further comprising a fourth step of obtaining a carboxylic acid anhydride containing not less than 0.99 of a carboxylic acid and not more than 0.002 of water from the top of the distillation column or the middle of the distillation column by re-distilling the carboxylic acid layer containing, in terms of mass fraction, less than 0.01 of water and less than 0.01 of a prepared extractant component, and distilling water and the prepared extractant component, the carboxylic acid layer being discharged by the third step.

15. The method according to any one of claims 2 to 12, further comprising a fifth step of distilling or stripping wastewater containing, in terms of mass fraction, less than 0.005 of a carboxylic acid, less than 0.001 of a prepared extractant component, and 0.003 to 0.2 of an inorganic salt in the aqueous solution discharged by the first step to distill the prepared extractant component and to obtain an inorganic salt aqueous solution, and returning the distilled prepared extractant component to a primary wastewater in the first step.

## Patentansprüche

1. Verfahren zur Rückgewinnung von Carbonsäure, mit einem Vorbehalt, dass die Carbonsäure von einer wässrigen Lösung getrennt ist, die Wasser und die Carbonsäure enthält und eine Carbonsäurekonzentration von 0,05 bis 0,3 gemessen als Massenfraktion aufweist, und wobei die getrennte Carbonsäure gereinigt ist und als ein Carbonsäureanhydrid enthalten wird, das weniger als 0,01 an Wasser und nicht weniger als 0,99 an Carbonsäure gemessen als Massenfraktion enthält, wobei das Verfahren Folgendes umfasst:
a) einen ersten Schritt, der einen Flüssig-flüssig-Extraktionsschritt umfasst, bei dem die wässrige Lösung in Flüssig-flüssig-Kontakt mit einem zubereiteten Extraktionsmittel gebracht wird, wobei das zubereitete Extraktionsmittel eine Komponente zum Extrahieren der Carbonsäure und ein Verdünnungsmittel zum Auflösen der Komponente enthält, wobei der Flüssig-flüssig-Kontakt in einem Zustand resultiert, in dem auf der Seite der wässrigen Lösung die Komponente des zubereiteten Extraktionsmittels in weniger als 0,001 gemessen als Massenfraktion aufgelöst ist und die Carbonsäurekonzentration in der wässrigen Lösung weniger als 0,005 gemessen als Massenfraktion beträgt, und auf der Seite des zubereiteten Extraktionsmittels die mit einer Rückgewinnungsrate von nicht weniger als 90 % aus der wässrigen Lösung getrennte Carbonsäure aufgelöst ist und Wasser in weniger als 0,05 gemessen als Massenfraktion aufgelöst ist;
b) einen zweiten Schritt, der einen Schritt des Destillierens des zubereiteten Extraktionsmittels umfasst, das die Carbonsäure und Wasser enthält, das Folgendem unterzogen wurde: dem ersten Schritt, Azeotropdestillation des Verdünnungsmittels in dem zubereiteten Extraktionsmittel und Wasser, Trennen des Destillats in eine Extraktionsmittelkomponentenschicht, die vorwiegend das Verdünnungsmittel enthält, und eine wässrige Schicht mit einem Dekantiergefäß, das in einer Destilliersäule bereitgestellt ist, und darauffolgendes Ablassen der wässrigen Schicht, Mischen des abgelassenen Wassers mit einer primären wässrigen Lösung oder einer sekundären wässrigen Lösung im ersten Schritt oder Entsorgen des abgelassenen Wassers als Abwasser, während die Extraktionsmittelkomponentenschicht, die vorwiegend das Verdünnungsmittel enthält, zum Destillationsschritt als Rückfluss zurückgeführt wird, und Ablassen des zubereiteten Extraktionsmittels, das die Carbonsäure enthält, durch einen Boden der Säule; und
c) einen dritten Schritt, der Folgendes umfasst: erneutes Destillieren des zubereiteten Extraktionsmittels, das die durch den zweiten Schritt abgelassene Carbonsäure enthält, und
in einem Fall, in dem kein Azeotrop zwischen der Carbonsäure und dem Verdünnungsmittel in dem zubereiteten Extraktionsmittel vorliegt, Ablassen durch einen oberen Teil der Säule einer gereinigten Carbonsäure, gemessen als Massenfraktion, das weniger als 0,01 an Wasser und weniger als 0,01 des zubereiteten Extraktionsmittels enthält, und Zurückführen des zubereiteten Extraktionsmittels, aus dem die Carbonsäure und Wasser entfernt wurde, zum ersten Schritt, wobei das zubereitete Extraktionsmittel durch den Boden der Säule abgelassen wird, oder
in einem Fall, in dem die Carbonsäure und das Verdünnungsmittel in dem zubereiteten Extraktionsmittel ein Siedepunktsminimumazeotrop aufweisen, Destillieren des Verdünnungsmittels und der Carbonsäure durch Azeotropdestillation, Trennen des Destillats in eine Extraktionsmittelkomponentenschicht, die vorwiegend das Verdünnungsmittel enthält, und eine Carbonsäureschicht mit einem Dekantiergefäß, das in einer Destilliersäule bereitgestellt ist, Ablassen der Carbonsäureschicht, die gemessen als Massenfraktion weniger als 0,01 an Wasser und weniger als 0,01 des zubereiteten Extraktionsmittels enthält, um die gereinigte Carbonsäure zu erhalten, während die Extraktionsmittelkomponentenschicht, die vorwiegend das Verdünnungsmittel enthält, als Rückfluss zum Destillationsschritt zurückgeführt wird, und Zurückführen des zubereiteten Extraktionsmittels, aus dem die Carbonsäure und Wasser entfernt wurden, zum ersten Schritt, wobei das zubereitete Extraktionsmittel durch einen Boden der Säule abgelassen wird,
wobei das Verdünnungsmittel in dem zubereiteten Extraktionsmittel ein hydrophobes Lösungsmittel ist, das einen Siedepunkt höher als den von Wasser und höher als den der Carbonsäure bei Umgebungsluftdruck aufweist, das **dadurch gekennzeichnet ist, dass** das Verdünnungsmittel ein Siedepunktsminimumazeotrop mit Wasser aufweist, wobei eine Wasserkonzentration in einer azeotropen Zusammensetzung mit dem Verdünnungsmittel nicht weniger ist als 0,2, gemessen als die Massenfraktion, und das Verdünnungsmittel kein Azeotrop mit der Carbonsäure aufweist, oder
wobei das Verdünnungsmittel in dem zubereiteten Extraktionsmittel ein hydrophobes Lösungsmittel ist, das einen Siedepunkt höher als den von Wasser und höher als den der Carbonsäure bei Umgebungsluftdruck aufweist, das **dadurch gekennzeichnet ist, dass** das Verdünnungsmittel ein Siedepunktsminimumazeotrop mit Wasser aufweist, wobei eine Wasserkonzentration in einer azeotropen Zusammensetzung mit dem Verdünnungsmittel nicht weniger ist als 0,2, gemessen als Massenfraktion, die Carbonsäure ein Siedepunktsminimumazeotrop mit dem Verdünnungsmittel aufweist, wobei die Carbonsäure und das Verdünnungsmittel in einem beliebigen Verhältnis in zwei Schichten getrennt sind und die Auflösung des Verdünnungsmittels in der Carbonsäure weniger ist als 0,002, gemessen als Massenfraktion, und
die Komponente zum Extrahieren der Carbonsäure ein schwer wasserlösliches organisches Lösungsmittel ist, das einen Siedepunkt höher als den des Verdünnungsmittels aufweist, ein Carbonsäure-Verteilungsverhältnis D von "Wasser" zu "der Komponente zum Extrahieren" (= Carbonsäure in der Komponente/Carbonsäure in Wasser) von nicht weniger als 0,3 aufweist und vollständig mit dem Verdünnungsmittel kompatibel ist.

2. Verfahren nach Anspruch 1, wobei die wässrige Lösung weiter ein anorganisches Salz in einem Massenfraktionsbereich von 0,003 bis 0,2 enthält und in dem Flüssig-flüssig-Extraktionsschritt im ersten Schritt das anorganische Salz in der wässrigen Lösung in dem zubereiteten Extraktionsmittel in einer Menge von weniger als 0,0001 und im Wasser in der wässrigen Lösung in einer Menge von 0,003 bis weniger als 0,2 aufgelöst ist.

3. Verfahren nach Anspruch 1 oder 2, wobei die in der wässrigen Lösung enthaltene Carbonsäure aus der Gruppe bestehend aus Ameisensäure, Essigsäure und Propionsäure ausgewählt ist.

4. Verfahren nach einem der Ansprüche 1 bis 3, wobei das in der wässrigen Lösung enthaltene anorganische Salz aus der Gruppe bestehend aus einem Metallchlorid, einem Metallsulfat, einem Metallhydrogensulfat, einem Metallhydroxid, einem Metallcarbonat, einem Metallhydrogencarbonat, einem Metallphosphat, einem Metallhydrogenphosphat und einem Metallborat ausgewählt ist.

5. Verfahren nach einem der Ansprüche 1 bis 4, wobei ein Siedepunkt des Verdünnungsmittels in dem zubereiteten Extraktionsmittel **110** bis 220 °C bei Umgebungsluftdruck beträgt und ein hydrophobes Lösungsmittel ist, das eine Löslichkeit in Wasser von weniger als 0,001 gemessen als Massenfraktion bei 25 °C aufweist, und wobei das Verdünnungsmittel vorzugsweise mindestens eines ist, ausgewählt aus der Gruppe bestehend aus Toluol, Octan, Isooctan, Nonan, Decan, Undecan, Dodecan, o-Xylol, m-Xylol, p-Xylol und Ethylbenzen.

6. Verfahren nach einem der Ansprüche 1 bis 5,
wobei die wässrige Lösung Unreinheiten enthält, die in der Carbonsäure gelöst sind und nicht in Wasser gelöst sind, und
vor Anwenden der wässrigen Lösung auf den ersten Schritt, ein Schritt des Entfernens der Unreinheiten durch Filtern der wässrigen Lösung durchgeführt wird oder die Carbonsäure in dem zubereiteten Extraktionsmittel im ersten Schritt wiedergewonnen wird, so dass die Unreinheiten, die an einer Schnittstelle mit einer Extraktionsmittelphase ausgefällt werden, aus einer Düse extrahiert werden, die in einem oberen Bereich oder einem Boden einer Flüssig-flüssig-Extraktionsvorrichtung bereitgestellt ist, zusammen mit dem zubereiteten Extraktionsmittel und der wässrigen Lösung, und die Unreinheiten durch Abtrennen einer Flüssigkeit, die die extrahierten Unreinheiten enthält, in Unreinheiten und eine Flüssigkeit durch Filtration oder Zentrifugation und Mischen einer wiedergewonnenen Flüssigkeit mit der primären wässrigen Lösung im ersten Schritt entfernt werden.

7. Verfahren nach einem der Ansprüche 1 bis 6, wobei die Komponente zum Extrahieren der Carbonsäure in dem zubereiteten Extraktionsmittel aus der Gruppe bestehend aus einer Organophosphat-Verbindung und einer Amid-Verbindung ausgewählt ist.

8. Verfahren nach einem der Ansprüche 1 bis 7, wobei ein Mischverhältnis der Komponente zum Extrahieren der Carbonsäure zu dem Verdünnungsmittel in dem zubereiteten Extraktionsmittel ein Verhältnis der Komponente zum Extrahieren der Carbonsäure : Verdünnungsmittel von 1:5 bis 9:1, gemessen als Massenverhältnis, ist.

9. Verfahren nach einem der Ansprüche 2 bis 8, wobei, wenn die wässrige Lösung, die das anorganische Salz enthält, dem ersten Schritt unterzogen wird, eine Flüssig-flüssig-Extraktionsvorrichtung bei einer Temperatur von 10 bis 90 °C in einem Zustand betrieben wird, in dem das anorganische Salz aufgelöst ist.

10. Verfahren nach einem der Ansprüche 1 bis 9, wobei der zweite Schritt und/oder der dritte Schritt bei einem Grad verringerten Drucks von 6,67 bis 66,7 kPa durchgeführt werden.

11. Verfahren nach einem der Ansprüche 2 bis 10, wobei ein Filterschritt einer Linie hinzugefügt wird, durch die eine Bodenflüssigkeit in den dritten Schritt übertragen wird, um das anorganische Salz zu entfernen, das an dem Boden der Destilliersäule durch Destillieren von Wasser aus dem zubereiteten Extraktionsmittel, das die Carbonsäure und Wasser im zweiten Schritt enthält, ausgefällt wird.

12. Verfahren nach einem der Ansprüche 1 bis **11,** wobei der zweite Schritt eine Linie einschließt, durch die das zubereitete Extraktionsmittel von einer beliebigen Wanne über einer Rohstoffzufuhrwanne getrennt von dem Rückfluss der Extraktionsmittelkomponentenschicht, die vorwiegend das Verdünnungsmittel enthält, zur Säule zugeführt wird, um eine Rückgewinnungsrate der Carbonsäure zu verbessern.

13. Verfahren nach einem der Ansprüche 1 bis 12, wobei der dritte Schritt eine Linie einschließt, durch die die Extraktionsmittelkomponentenschicht, die vorwiegend ein Verdünnungsmittel enthält, von einer beliebigen Wanne unter einer Rohstoffzufuhrwanne getrennt von dem Rückfluss der Extraktionsmittelkomponentenschicht, die vorwiegend das Verdünnungsmittel enthält, zu der Säule zugeführt wird, um eine Rückgewinnungsrate der Carbonsäure zu verbessern.

14. Verfahren nach einem der Ansprüche 1 bis 13, weiter umfassend einen vierten Schritt des Erhaltens eines Carbonsäureanhydrids, das nicht weniger als 0,99 einer Carbonsäure und nicht mehr als 0,002 Wasser aus dem oberen Bereich der Destilliersäule oder der Mitte der Destilliersäule enthält, durch erneutes Destillieren der Carbonsäureschicht, die, gemessen als Massenfraktion, weniger als 0,01 Wasser und weniger als 0,01 einer zubereiteten Extraktionsmittelkomponente enthält, und Destillieren von Wasser und der zubereiteten Extraktionsmittelkomponente, wobei die Carbonsäureschicht durch den dritten Schritt abgelassen wird.

15. Verfahren nach einem der Ansprüche 2 bis 12, weiter umfassend einen fünften Schritt des Destillierens oder Abtriebs von Abwasser, das, gemessen als Massenfraktion, weniger als 0,005 einer Carbonsäure, weniger als 0,001 einer zubereiteten Extraktionsmittelkomponente und 0,003 bis 0,2 eines anorganischen Salzes in der wässrigen Lösung enthält, die durch den ersten Schritt abgelassen wurde, um die zubereitete Extraktionsmittelkomponente zu destillieren und eine ein anorganisches Salz enthaltende wässrige Lösung zu erhalten, und Zurückführen der destillierten zubereiteten Extraktionsmittelkomponente zu einem primären Abwasser im ersten Schritt.

## Revendications

1. Procédé de collecte d'un acide carboxylique, à condition que l'acide carboxylique soit séparé d'une solution aqueuse contenant de l'eau et l'acide carboxylique et présentant une concentration en acide carboxylique de 0,05 à 0,3 en matière de fraction massique et que l'acide carboxylique séparé soit purifié et obtenu sous forme d'anhydride d'acide carboxylique contenant moins de 0,01 d'eau et pas moins de 0,99 d'acide carboxylique en matière de fraction massique, le procédé comprenant :
a) une première étape comprenant une étape d'extraction liquide-liquide consistant à mettre la solution aqueuse en contact liquide-liquide avec un agent d'extraction préparé, l'agent d'extraction préparé contenant un composant pour extraire l'acide carboxylique et un diluant pour dissoudre le composant, le contact liquide-liquide entraînant un état dans lequel, côté solution aqueuse, le composant de l'agent d'extraction préparé est dissous à moins de 0,001 en matière de fraction massique et la concentration en acide carboxylique dans la solution aqueuse est inférieure à 0,005 en matière de fraction massique, et, côté agent d'extraction préparé, l'acide carboxylique séparé avec un taux de récupération de pas moins de 90 % à partir de la solution aqueuse est dissous et de l'eau étant dissoute à moins de 0,05 en matière de fraction massique ;
b) une deuxième étape comprenant une étape de distillation de l'agent d'extraction préparé contenant l'acide carboxylique et l'eau qui est soumis à la première étape, en effectuant une distillation azéotropique du diluant dans l'agent d'extraction préparé et de l'eau, la séparation du distillat en une phase de composant d'agent d'extraction contenant principalement le diluant et en une phase aqueuse avec un décanteur équipant une colonne de distillation, puis l'évacuation de la phase aqueuse, le mélange de l'eau évacuée avec une solution aqueuse primaire ou une solution aqueuse secondaire dans la première étape ou l'élimination de l'eau évacuée en tant qu'eau usée tout en renvoyant la phase de composant d'agent d'extraction contenant principalement le diluant vers l'étape de distillation sous forme de reflux, et l'évacuation de l'agent d'extraction préparé contenant l'acide carboxylique par le bas de la colonne ; et
c) une troisième étape comprenant la redistillation de l'agent d'extraction préparé contenant l'acide carboxylique évacué par la deuxième étape, et
dans le cas où il n'y a pas d'azéotrope entre l'acide carboxylique et le diluant dans l'agent d'extraction préparé, l'évacuation, par le haut de la colonne, d'un acide carboxylique purifié contenant, en matière de fraction massique, moins de 0,01 d'eau et moins de 0,01 de l'agent d'extraction préparé, et le renvoi, vers la première étape, de l'agent d'extraction préparé duquel l'acide carboxylique et l'eau sont retirés, l'agent d'extraction préparé étant évacué par le bas de la colonne, ou
dans un cas où l'acide carboxylique et le diluant dans l'agent d'extraction préparé présentent un azéotrope à ébullition minimale, la distillation du diluant et de l'acide carboxylique par distillation azéotropique, la séparation du distillat en une phase de composant d'agent d'extraction contenant principalement le diluant et en une phase d'acide carboxylique avec un décanteur équipant une colonne de distillation, l'évacuation de la phase d'acide carboxylique contenant, en matière de fraction massique, moins de 0,01 d'eau et moins de 0,01 de l'agent d'extraction préparé pour obtenir l'acide carboxylique purifié tout en renvoyant la phase de composant d'agent d'extraction contenant principalement le diluant vers l'étape de distillation sous forme de reflux, et le renvoi, vers la première étape, de l'agent d'extraction préparé duquel l'acide carboxylique et l'eau sont retirés, l'agent d'extraction préparé étant évacué par le bas de la colonne,
le diluant dans l'agent d'extraction préparé étant un solvant hydrophobe présentant un point d'ébullition supérieur à celui de l'eau et supérieur à celui de l'acide carboxylique à pression atmosphérique, qui est **caractérisé en ce que** le diluant présente un azéotrope à ébullition minimale avec l'eau, une concentration en eau dans une composition azéotropique avec le diluant n'est pas inférieure à 0,2 en matière de fraction massique, et le diluant ne présente pas d'azéotrope avec l'acide carboxylique, ou
le diluant dans l'agent d'extraction préparé étant un solvant hydrophobe présentant un point d'ébullition supérieur à celui de l'eau et supérieur à celui de l'acide carboxylique à pression atmosphérique, qui est **caractérisé en ce que** le diluant présente un azéotrope à ébullition minimale avec l'eau, une concentration en eau dans une composition azéotropique avec le diluant n'est pas inférieure à 0,2 en matière de fraction massique, l'acide carboxylique présente un azéotrope à ébullition minimale avec le diluant, l'acide carboxylique et le diluant sont séparés en phases à n'importe quel rapport, et la dissolution du diluant dans l'acide carboxylique est inférieure à 0,002 en matière de fraction massique, et
le composant pour extraire l'acide carboxylique étant un solvant organique peu soluble dans l'eau qui présente un point d'ébullition supérieur à celui du diluant, présente un rapport D de distribution de l'acide carboxylique entre « l'eau » et « le composant d'extraction » (= l'acide carboxylique dans le composant d'extraction/l'acide carboxylique dans l'eau) de pas moins de 0,3, et est entièrement compatible avec le diluant.

2. Procédé selon la revendication 1, dans lequel la solution aqueuse contient en outre un sel inorganique dans une plage de fraction massique de 0,003 à 0,2, et dans l'étape d'extraction liquide-liquide dans la première étape, le sel inorganique dans la solution aqueuse est dissous dans l'agent d'extraction préparé en une quantité inférieure à 0,0001 et dans l'eau dans la solution aqueuse en une quantité de 0,003 à moins de 0,2.

3. Procédé selon la revendication 1 ou la revendication 2, dans lequel l'acide carboxylique contenu dans la solution aqueuse est choisi dans le groupe consistant en l'acide formique, l'acide acétique et l'acide propionique.

4. Procédé selon l'une quelconque des revendications 1 à 3, dans lequel le sel inorganique contenu dans la solution aqueuse est choisi dans le groupe consistant en un chlorure métallique, un sulfate métallique, un hydrogénosulfate métallique, un hydroxyde métallique, un carbonate métallique, un hydrogénocarbonate métallique, un phosphate métallique, un hydrogénophosphate métallique, et un borate métallique.

5. Procédé selon l'une quelconque des revendications 1 à 4, dans lequel un point d'ébullition du diluant dans l'agent d'extraction préparé est de **110** à 220 °C à pression atmosphérique, et est un solvant hydrophobe présentant une solubilité dans l'eau inférieure à 0,001 en matière de fraction massique à 25 °C, et dans lequel le diluant est de préférence au moins un sélectionné dans le groupe consistant en le toluène, l'octane, l'isooctane, le nonane, le décane, l'undécane, le dodécane, l'o-xylène, le m-xylène, le p-xylène et l'éthylbenzène.

6. Procédé selon l'une quelconque des revendications 1 à 5,
dans lequel la solution aqueuse contient des impuretés qui sont dissoutes dans l'acide carboxylique et ne sont pas dissoutes dans l'eau, et
avant l'application de la solution aqueuse à la première étape, une étape d'élimination des impuretés par filtration de la solution aqueuse est réalisée, ou l'acide carboxylique est collecté dans l'agent d'extraction préparé à la première étape, de sorte que les impuretés qui précipitent à une interface avec une phase d'agent d'extraction sont extraites à partir d'une buse prévue en haut ou en bas d'un dispositif d'extraction liquide-liquide conjointement avec l'agent d'extraction préparé et la solution aqueuse, et les impuretés sont éliminées en séparant un liquide contenant les impuretés extraites en impuretés et en un liquide par filtration ou centrifugation et en mélangeant un liquide collecté avec la solution aqueuse primaire dans la première étape.

7. Procédé selon l'une quelconque des revendications 1 à 6, dans lequel le composant pour extraire l'acide carboxylique dans l'agent d'extraction préparé est choisi dans le groupe consistant en un composé organophosphoré et un composé d'amide.

8. Procédé selon l'une quelconque des revendications 1 à 7, dans lequel un rapport de mélange du composant pour extraire l'acide carboxylique par rapport au diluant dans l'agent d'extraction préparé est un rapport composant pour extraire l'acide carboxylique: diluant de 1:5 à 9:1 en matière de rapport massique.

9. Procédé selon l'une quelconque des revendications 2 à 8, dans lequel lorsque la solution aqueuse contenant le sel inorganique est soumise à la première étape, un dispositif d'extraction liquide-liquide est utilisé à une température de 10 à 90 °C dans un état où le sel inorganique est dissous.

10. Procédé selon l'une quelconque des revendications 1 à 9, dans lequel la deuxième étape et/ou la troisième étape sont réalisées à un degré de réduction de pression de 6,67 à 66,7 kPa.

11. Procédé selon l'une quelconque des revendications 2 à 10, dans lequel une étape de filtration est ajoutée à une ligne par laquelle un liquide de fond est transféré dans la troisième étape pour éliminer le sel inorganique qui précipite au fond de la colonne de distillation en distillant l'eau à partir de l'agent d'extraction préparé contenant l'acide carboxylique et de l'eau dans la deuxième étape.

12. Procédé selon l'une quelconque des revendications 1 à **11,** dans lequel la deuxième étape inclut une ligne par laquelle l'agent d'extraction préparé est fourni à la colonne à partir d'un plateau arbitraire au-dessus d'un plateau d'alimentation en matière première, séparément du reflux de la phase de composant d'agent d'extraction contenant principalement le diluant, pour améliorer un taux de récupération de l'acide carboxylique.

13. Procédé selon l'une quelconque des revendications 1 à 12, dans lequel la troisième étape inclut une ligne par laquelle la phase de composant d'agent d'extraction contenant principalement un diluant est fournie à la colonne à partir d'un plateau arbitraire sous un plateau d'alimentation en matière première, séparément du reflux de la phase de composant d'agent d'extraction contenant principalement le diluant, pour améliorer un taux de récupération de l'acide carboxylique.

14. Procédé selon l'une quelconque des revendications 1 à 13, comprenant en outre une quatrième étape d'obtention d'un anhydride d'acide carboxylique contenant pas moins de 0,99 d'acide carboxylique et pas plus de 0,002 d'eau à partir du haut de la colonne de distillation ou du milieu de la colonne de distillation par redistillation de la phase d'acide carboxylique contenant, en matière de fraction massique, moins de 0,01 d'eau et moins de 0,01 d'un composant d'agent d'extraction préparé, et distillation de l'eau et du composant d'agent d'extraction préparé, la phase d'acide carboxylique étant évacuée par la troisième étape.

15. Procédé selon l'une quelconque des revendications 2 à 12, comprenant en outre une cinquième étape de distillation ou d'extraction d'eau usée contenant, en matière de fraction massique, moins de 0,005 d'un acide carboxylique, moins de 0,001 d'un composant d'agent d'extraction préparé, et 0,003 à 0,2 d'un sel inorganique dans la solution aqueuse évacuée lors de la première étape pour distiller le composant d'agent d'extraction préparé et pour obtenir une solution aqueuse de sel inorganique, et le renvoi du composant d'agent d'extraction préparé distillé dans une eau usée primaire dans la première étape.
